(19)

**Europäisches Patentamt**
**European Patent Office**
**Office européen des brevets**

(11) **EP 4 687 143 A1**

(12) **EUROPEAN PATENT APPLICATION**

(43) Date of publication:
**04.02.2026 Bulletin 2026/06**

(21) Application number: **24191385.4**

(22) Date of filing: **29.07.2024**

(51) International Patent Classification (IPC):
**G16B 40/20** (2019.01) **G16H 30/40** (2018.01)
**G16H 50/20** (2018.01)

(52) Cooperative Patent Classification (CPC):
**G16H 50/20; G16B 40/20; G16H 30/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Fujitsu Limited**
**Kawasaki-shi, Kanagawa 211-8588 (JP)**

(72) Inventor: **AMER, Mohammed**
**Slough, SL1 2BE (GB)**

(74) Representative: **Haseltine Lake Kempner LLP**
**Cheapside House**
**138 Cheapside**
**London EC2V 6BJ (GB)**

Remarks:
Amended claims in accordance with Rule 137(2) EPC.

(54) **BREAST CANCER CLASSIFICATION**

(57) A computer-implemented method comprising: applying a first machine learning, ML, model on copy number variation, CNV, data relating to a subject to generate first output logits corresponding respectively to a plurality of breast cancer sub-types; applying a second ML model on histopathology image data relating to the subject to generate second output logits corresponding respectively to the plurality of breast cancer sub-types; applying a third ML model on medical data relating to the subject, including information relating to a diagnosis of breast cancer in the subject, to generate third output logits corresponding respectively to the plurality of breast cancer sub-types; computing a weighted combination of the first output logits, the second output logits, and the third output logits; and based on the weighted combination, classifying the subject's breast cancer as a sub-type of the plurality of breast cancer sub-types.

FIG. 1

**EP 4 687 143 A1**

**Description**

The present invention relates to breast cancer classification, and in particular to a computer-implemented method, a computer program, and an information programming apparatus.

Breast cancer is the most common diagnosed cancer among women worldwide. However, diagnosing a patient simply with Breast cancer is a strong simplification since Breast cancer exhibits genetic and clinical heterogeneity that affects the prognosis and treatment plans.

Cancers are heterogeneous diseases that present with genetic and molecular diversity. Classifying cancers into more granular subtypes can help with treatment plan tailoring which can improve patient prognosis. The PAM50 Breast cancer subtype classification is a widely used genetic classification based on 50-genes RNA analysis of the tumour that is practically used for prognosis prediction and treatment tailoring. However, RNA profiling is an expensive and difficult process that is not always a regular procedure in clinical setting.

In light of the above, a breast cancer classification method is desired.

The present invention is defined by the independent claims, to which reference should now be made. Specific embodiments are defined in the dependent claims.

According to an embodiment of a first aspect there is disclosed herein a computer-implemented method comprising: applying a first machine learning, ML, model on copy number variation, CNV, data relating to a subject to generate first output logits corresponding respectively to a plurality of breast cancer sub-types, wherein the first ML model has been trained to classify CNV data as corresponding to a sub-type of the plurality of breast cancer sub-types; applying a second ML model on histopathology image data relating to the subject to generate second output logits corresponding respectively to the plurality of breast cancer sub-types, wherein the second ML model has been trained to classify histopathology data as corresponding to a sub-type of the plurality of breast cancer sub-types; applying a third ML model on medical data relating to the subject, including information relating to a diagnosis of breast cancer in the subject, to generate third output logits corresponding respectively to the plurality of breast cancer sub-types, wherein the third ML model has been trained to classify medical data, including information relating to a diagnosis of breast cancer, as corresponding to a sub-type of the plurality of breast cancer sub-types; computing a weighted combination of the first output logits, the second output logits, and the third output logits; and based on the weighted combination, classifying the subject's breast cancer as a sub-type of the plurality of breast cancer sub-types.

Reference will now be made, by way of example, to the accompanying drawings, in which:

Figure 1 is a diagram of a system;
Figure 2 is a diagram of a system overview;
Figure 3 is a diagram of a system overview;
Figure 4 is a diagram indicating training data;
Figure 5 is a diagram illustrating an SELU;
Figure 6 is a diagram illustrating a first ML model architecture;
Figure 7 is a diagram useful for understanding the second ML model;
Figure 8 is a diagram illustrating a second ML model architecture;
Figure 9 is a diagram illustrating a third ML model architecture;
Figure 10 is a diagram illustrating a method;
Figure 11 is a diagram illustrating results of a worked example;
Figure 12 is a diagram illustrating results of a worked example;
Figure 13 is a diagram illustrating a WSI of a worked example;
Figure 14 is a diagram illustrating a WSI of a worked example;
Figure 15 is a diagram useful for understanding the second ML model;
Figure 16 is a diagram illustrating results of a worked example;
Figure 17 is a diagram useful for understanding the second ML model;
Figure 18 is a diagram illustrating results of a worked example;
Figure 19 is a diagram illustrating results of a worked example;
Figure 20 is a diagram illustrating results of a worked example;
Figure 21 is a diagram illustrating results of a worked example;
Figure 22 is a diagram illustrating results of a worked example; and
Figure 23 is a diagram illustrating a computing device.

The following definitions may be used in the description but are not exhaustive.

*Copy Number Variation (CNV)* - Copy Number Variation is a quantification of the variation, repeats and duplication that affect a considerable number of base pairs of the DNA.

*Electronic Health Records (EHR)* - Electronic Health Records are the collection of patient clinical data in a digital format. This includes information like medical history, medications, lab tests and demographics.

*Whole-Slide Image (WSI)* - A Whole-Slide Image is a digital high-resolution scan of a histopathology slide. WSIs are usually muti-gigapixel images at the highest resolution stored with optionally down-sampled versions.

*Ribonucleic Acid (RNA)* - RNA is a polymeric molecule that is essential to cellular functions. It can be functional itself (e.g some non-coding RNA) or acts as a template for protein synthesis.

*RNA-Seq* - RNA Sequencing is a technique that uses next-generation sequencing to provide a snapshot of the gene expression or transcriptome in a tissue sample.

*Convolutional Neural Network (CNN)* - A CNN is a neural network model that utilizes linear filter combined with non-linear activations to perform a Machine Learning task. It is most commonly used in Computer Vision applications.

*Support Vector Machine (SVM)* - An SVM is a classical supervised Machine Learning model that can be used for classification and regression. It is motivated by finding the maximal-margin hyperplane.

*Circulating Tumour DNA (ctDNA)* - ctDNA refers to fragmented DNA in the blood stream that is not associated with a cell and has a tumour origin.

*Tumour Micro-Environment (TME)* - TME refers to the complex ecosystem and cellular composition that surrounds the tumour and affects its behaviour.

*Fusion* - In Machine learning, fusion refers to integrating multiple sources of data to perform some downstream task. It can generally classified into Intermediate Fusion and Late Fusion. Intermediate Fusion is based on integrating intermediate representations from multiple models to perform the downstream task. On the other hand, Late Fusion depends on the output of independent models.

*PAM50* - PAM50 is a breast cancer molecular subtyping method based on a 50-genes signature. Usually it has five subtypes: Luminal A, Luminal B, Her2-enriched, Basal-like and Normal-like. Often, only four of the subtypes are used (normal-like is omitted).

*Self-Normalizing Network (SNN)* - A Self-Normalizing Network is a feedforward fully-connected neural network that is designed to preserve activation normalization, which can help with depth and high-dimensional low sample size data.

*Integrated gradients (IG)* - Integrated Gradients is an input attribution and explainability method for differentiable Machine Learning models. It is based on taking the path integral of the gradient from a baseline to the input.

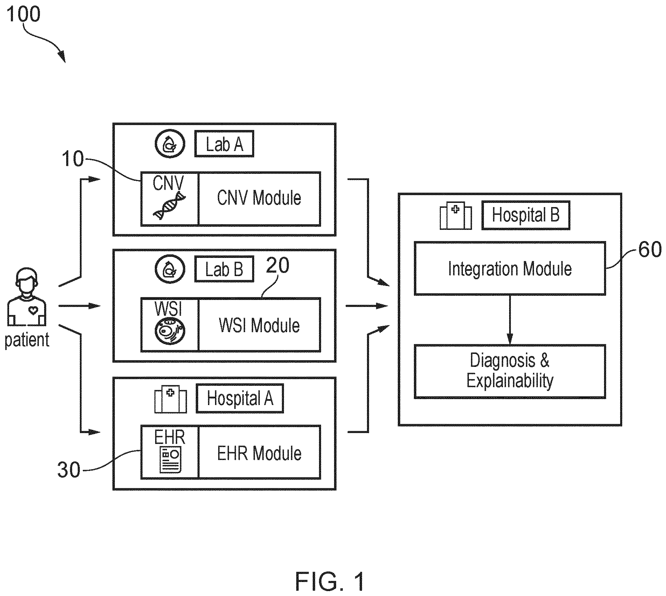

Figure 1 is schematic diagram of a system 100 comprising a CNV module 10, a WSI module 20, an EHR module 30, and an integration module 60. The modules 10, 20, 30 each apply a machine learning (ML) model on a corresponding type of data related to a subject. The ML models are for classifying the type of data concerned as corresponding to a sub-type of a plurality of breast cancer sub-types (e.g. the PAM50 sub-types). The modules 10, 20, 30 provide output logits indicating their prediction to the integration module 60, which performs late fusion and based on the output logits classifies the breast cancer of the subject as a sub-type of the plurality of breast cancer sub-types.

The CNV Module 10 is responsible for the pre-processing and modality-specific prediction for CNV data. After pre-processing the CNV data, the module 10 extracts features from the preprocessed data. This is followed by providing prediction and explainability for the data. The module 10 provides indication of the attribution of the different input genes that shows the importance of each for the module's 10 decision.

The WSI module 20 is responsible for the processing of WSI data. WSIs are multi-gigapixel images that are difficult (time- and/or resource-expensive) to process as a single unit. This module 20 is responsible for segmentation and tile extraction from WSIs to make it feasible to apply a neural network (the ML model concerned) to the data. After dividing the WSI into smaller patches, the module 20 is responsible for filtering empty patches based on tissue content. The module 20 operates for example on 20x down-sampled patches which help to make it computationally efficient. After the preprocessing and feature extraction, the WSI module 20 produces a prediction for each patch. The module 20 is responsible for aggregating patch predictions into a single prediction for a given WSI (and thus for a given subject) using majority voting. The module 20 provides explainability for the WSI as a heatmap that shows which regions are important for the model decision.

The EHR module 30 is responsible for the local processing and prediction for clinical health records. EHR features can be categorized into numerical (e.g Diagnosis age), ordinal (e.g IHC (Immunohistochemistry) score) and categorical (e.g ICD (International Classification of Diseases)-10 classification). The module 30 is responsible for preprocessing and conversion of ordinal and categorical into numerical format. It is also responsible for the normalization of features before applying the prediction model. The module 30 produces clinical data-specific subtype prediction and provides feature attribution that shows the contribution of clinical features to the prediction.

The Integration Module 60 carries out late fusion to integrate the distributed module 10, 20, 30 outputs into a multi-modal prediction. Each module 10, 20, 30 produces logits that encode a probability prediction for the four PAM50 classes. The module outputs are serialized and transferred to the integration module 60. The integration module 60 assigns a weight for each modality and for each class (sub-type) logit. The module 60 then integrates the logits by weighting each modality and each class output and sums the contribution from all modules 10, 20, 30. This is followed by a Softmax function that normalizes the logits into a valid probability distribution that encodes the PAM50 classification probability.

The modules 10, 20, 30, 60 are able to operate in a distributed manner on local data without the need for central data collection. Moreover, they can operate in a heterogeneous environment that can utilize CPU or GPU processing based on the workload of the modality. There is no dependency on RNA sequencing which makes the system feasible in clinical settings. The distributed nature of the system 100 is supported by the integration module 60 depending on the module 10, 20, 30 outputs (logits), not necessarily the raw data. The system 100 is able to provide a high performance for PAM50 breast cancer subtype classification. The WSI module 20 is able to operate on low-magnification 20x down-sampled patches which makes it resource efficient. Finally, each module 10, 20, 30 is able to provide explainability for its modality inputs which assists in understanding its decision and provides transparency for the system user.

The distributed nature of the system 100 is illustrated in Figure 1 in that the modules 10, 20, 30, 60 are located in different physical locations, e.g. in different labs and hospitals. For example, the modules may be located in locations where the data needed is stored, and thus the communication of data can be minimized/avoided. That is, patient data may reside on different systems and in different places. Single modality modules (CNV, WSI or EHR) may be deployed independently on different locations to process local data. Each module may provide a modality-specific output and explainability for its modality. The integration module 60 is responsible for integrating modality-specific model outputs to provide the multi-modal prediction for the patient/subject.

Each module illustrated in Figure 1 may be considered at least one processor configured to carry out the above-described functions of the module. At least one processor may carry out the operations of all the modules.

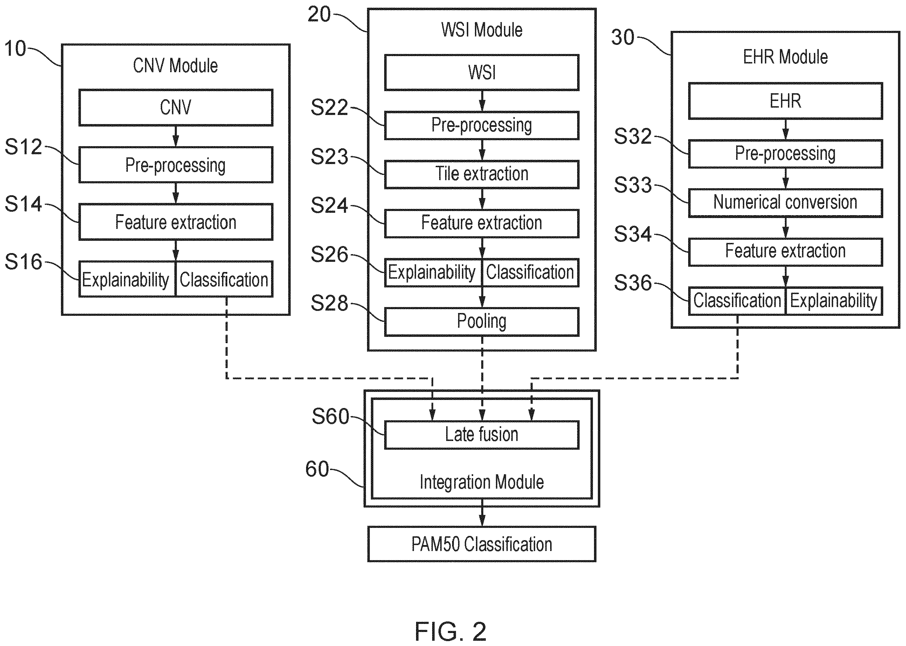

Figure 2 is a diagram illustrating method steps/operations performed by each of the modules 10, 20, 30 60 in an implementation. Each single-modality module 10, 20, 30 performs a pre-processing step S12, S22, S32 to pre-process the data. In the WSI module step S23 is performed to divide the WSI into tiles and extract relevant tiles. In the EHR module step S33 is performed to convert ordinal and categorical features to numerical features. In each module 10, 20, 30, feature extraction (S14, S24, S34) is performed to extract features from the data concerned and a classification step (S16, S26, S36) is performed to predict a sub-type for the subject's breast cancer. In the WSI module the classification is carried out on a per-tile basis, and so a pooling step (S28) follows to pool the per-tile predictions. The output logits from each module 10, 20, 30 are used by the integration module 60 in a late fusion step S60 to predict a PAM50 classification for the subject's breast cancer.

Training for the system 100 is done in two stages. First, each modality-specific ML model is trained on its modality using classification targets. After convergence, the weights of the single modality ML models are frozen. Then, using the modality-specific model outputs, weights used in the integration module 60 are trained on the same classification targets.

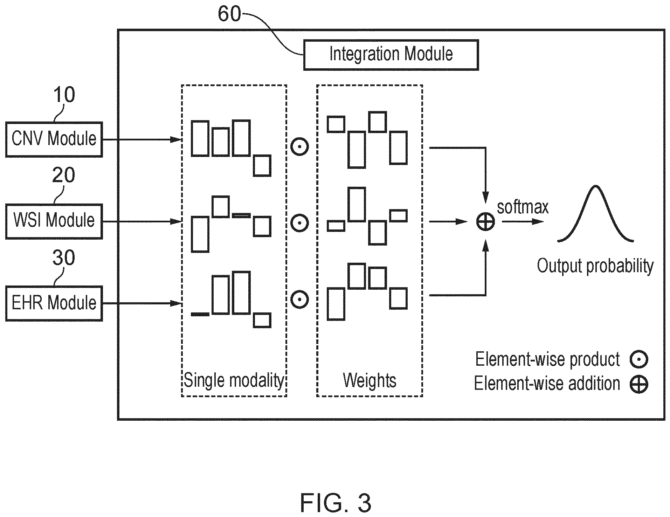

Figure 3 is a diagram illustrating an overview representative of functions of the modules 10, 20, 30, and 60. The modules 10, 20, 30 each carry out a single-modality prediction and output logits resulting therefrom are used in the integration module 60 to generate a multi-modality prediction.

As mentioned above, the modules 10, 20, 30, 60 are not essential but provide a useful division of operations for description. The processing of the CNV data (i.e. operations of the CNV module 10) may be considered to use a first ML model, the processing of the WSI data (i.e. operations of the WSI module 20) a second ML model, and the processing of the EHR data (i.e. operations of the EHR module 30) a third ML model. The processing of the CNV, WSI, and EHR data and the first-third ML models is described according to an implementation example below.

CNV Data Processing

CNV data is a quantification of duplication and deletion in DNA segments. For each gene, CNV provides a discrete value that describes this information:

- Deep deletion: -2
- Shallow deletion: -1
- Diploid: 0
- Gain: 1
- Amplification: 2

A value of zero means no repetition or deletion, positive values indicate repetition while negative values indicate deletion. Below is a random sample of 15 genes taken from the XXXXXX dataset (referred to later below) and their indices.

gene - index:

MYL12B - 123120
LINC00923 - 9657
RN7SKP161 - 16052
GBA - 6582
DEDD - 4467

RN7SL435P - 16560
CEBPG - 3144
ZSWIM5 - 23270
DCTN1 - 4386
MRPL4 - 12026
C9orf142 - 2357
LINC00842 - 9602
NAA20 - 12388
DTX4 - 4929
PTPRO - 15351

Preprocessing includes removing genes with missing CNV values. Preprocessing further includes z-scoring the CNV values using the mean and standard deviation of the training set (which the first ML model was trained on) according to:

$$z = (x - \mu) / \sigma$$

where x is the input, $\mu$ is the mean and $\sigma$ is the standard deviation.

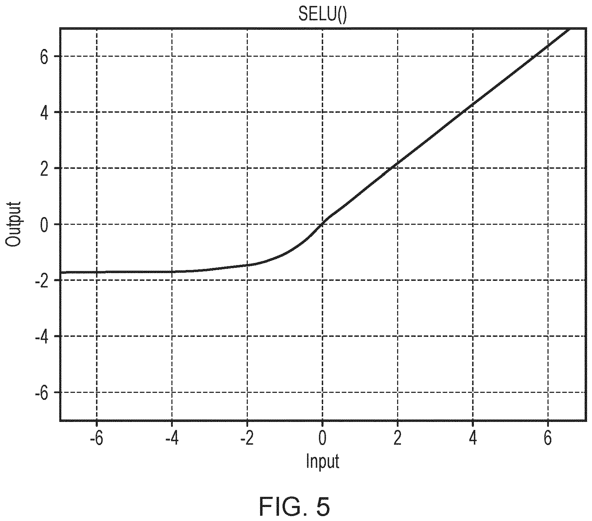

The first ML model comprises a Self-Normalizing Network (SNN). An SNN is a feedforward fully-connected network which is designed to preserve activation normalization. This may help with depth and high-dimensional low-size sample data like that sometimes common in CNV data. An SNN differs from a standard feedforward network in two ways. First, it uses Scaled-Exponential Linear Units (SELU) as the activation function (Figure 5 is a graph illustrating the Scaled-Exponential Linear Unit). Second, it uses a variation of Dropout called Alpha-Dropout which is more suitable for SELU activations and is able to preserve self-normalization.

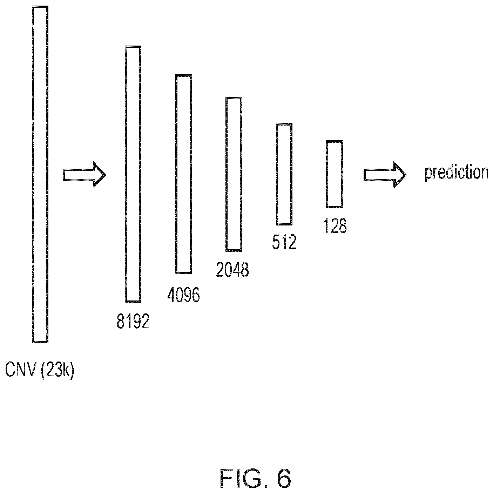

Figure 6 illustrates a specific architecture used in a specific implementation example of the first ML model. This architecture comprises an SNN with 5 hidden layers having 8192, 4096, 2048, 512 and 128 channels. The CNV data in this specific implementation example comprises 23k records.

To provide explainability for the CNV data processing, Integrated Gradients (IG) may be used. IG is a machine learning explainability methodology that assigns attribution to each feature in the input by accumulating the prediction gradient along a path integral from a baseline to the input. IG may be used to get the attribution score for each gene in the input. The absolute value of the attribution score may be used to rank genes based on their contribution to the first ML model's decision. This provides the system user with the importance of each gene for the model prediction which is useful for explainability.

CNV data may be considered a genetic modality. Another genetic modality that may be used is RNA-seq. A first auxiliary ML model having the same architecture as the first ML model may be used to generate output logits based on RNA-seq data. Pre-processing of RNA-seq data may comprise removing genes with missing values and transforming the data using a shifted Log2. The first auxiliary ML model may be considered implemented by the CNV module 10 or a separate module, e.g. an RNA module, similar to the modules 10, 20, 30. The integration module 60 may include the output logits from RNA-seq processing (the first auxiliary ML model) in its processing.

WSI Data Processing

A WSI is usually a multi-gigapixel image of a tissue sample. There are multiple challenges in using WSI for machine learning. Due to the large size and resolution, feeding the whole WSI into a machine learning model in a single pass may be infeasible due to memory and storage constraints. Another complication is that usually a large area of the WSI is background that does not contribute information to the learning process.

Pre-processing the WSI data comprises dividing the WSI into non-overlapping patches. After downsampling each patch, the tissue is segmented from the background by 1) converting the patch into Hue-Saturation-Value (HSV) color space, 2) applying median blurring to the saturation channel (e.g. using a kernel size of 7) and 3) generating a tissue mask by thresholding the saturation channel using a binary threshold (e.g. of 20 (for 0-255 pixel values)). Then, based on the generated mask, patches are selected only if the area of the tissue content is more than 5%.

Put another way, WSIs are digitized multi-gigapixel images of histopathology. Since processing the whole WSI raw would be time- and resource-expensive, the WSI is first tiled into smaller patches that can be processed individually by a neural network in the second ML model. However, a large area of the WSI is usually a background, which contributes no information to the prediction process and only adds computational complexity. Therefore patches that do not contain enough information are removed ads described above.

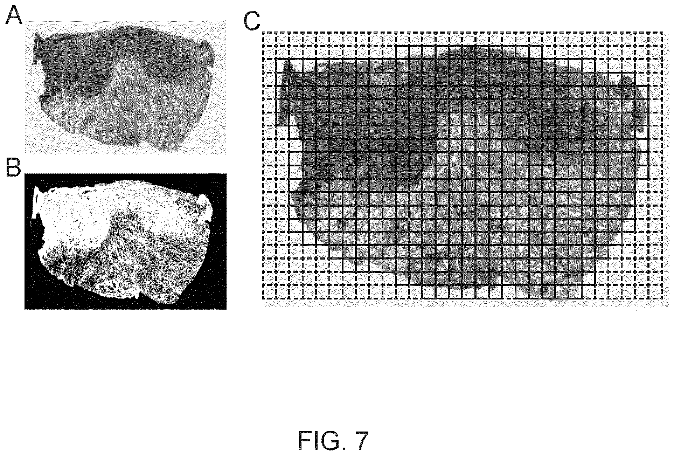

An example of preprocessing a WSI is shown in Figure 7. "A" shows an example input WSI (shown in black and white but in practice it is colored). First, the tissue content is separated from the background using the following segmentation algorithm:

- The WSI is tiled into (e.g. 256x256) non-overlapping patches
- The image in converted from RGB to HSV colour space. HSV decomposes the colour-space into Hue, Saturation and Value or Brightness.
- The saturation channel is filtered using median blurring. Median blurring uses a moving window or a kernel where the target pixel is replaced by the median in the window. A kernel size of 7 may be used.
- A mask is generated by thresholding the filtered saturation channel. A binary threshold with a threshold of 20 for pixel values ranging from 0 to 255 may be used.

An example generated mask is shown in (B). The patches are then filtered based on tissue content. The mask is used to determine the fractional area of each patch occupied by the tissue. A patch is accepted only if it has tissue fraction above a certain threshold (e.g. a threshold of 5%). Accepted patches are then (e.g. 20x) down-sampled to reduce computational complexity. (C) illustrates the patch filtration process - patches indicated with solid lines are accepted while patches indicated with broken lines are rejected. The preprocessing further includes that each patch is transformed as following:

- The patch may be resized (e.g. into 342x342) using bilinear interpolation
- A central crop (e.g. of 299x299) may be applied.
- The pixel values may be rescaled to [0.0, 1.0] (i.e. rescaled to be between 0 and 1) and normalized using mean and standard deviation of the training WSI data.

The second ML model may comprise a neural network/fully-connected neural network/convolutional neural network. In a specific implementation example, the second ML model comprises a classification head on top of a backbone of a pretrained computer vision model (e.g. Inceptionv3 (Szegedy, C., et al (2016), Rethinking the inception architecture for computer vision, In Proceedings of the IEEE conference on computer vision and pattern recognition (pp. 2818-2826)), VGG16 (K. Simonyan and A. Zisserman, Very deep convolutional networks for large-scale image recognition, arXiv preprint arXiv:1409.1556 (2014)) and Dinov2 (M. Oquab, et al., Dinov2: Learning robust visual features without supervision, arXiv preprint arXiv:2304.07193 (2023))).

Each subject/patient may have multiple WSIs and each WSI contributes multiple patches. The ground truth labels for the downstream task are patient-wise. Therefore a pooling strategy is used to convert patch-level predictions into patient-level predictions. For example two general pooling strategies include one based on pooling the classifier output and another based on pooling intermediate representations. For pooling the output, the following examples could be used: majority voting, mean of the output logits and mean of the output probabilities. For pooling intermediate representations, the following examples could be used: using either the mean or weighted average using distance from the mean.

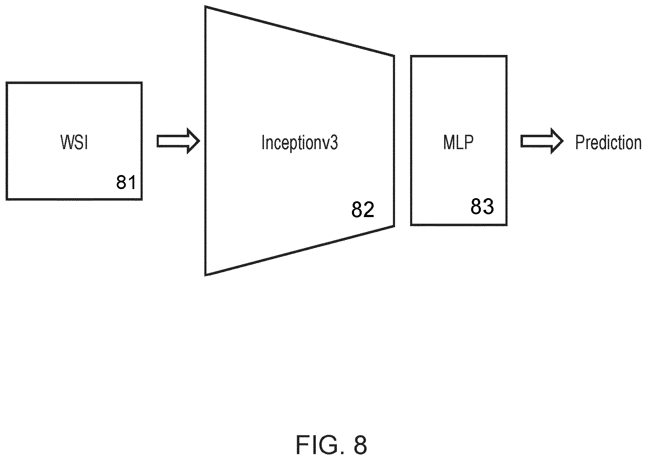

Figure 8 illustrates the architecture of the second ML model according to a specific implementation example, where the second ML model comprises a classification head (an MLP 83 in this case) on top of an Inceptionv3 backbone 82, and accepts as input WSI data 81.

Explainability for the second ML model may be provided as a heatmap using Integrated Gradients. IG may be used to calculate the attribution from each pixel in each accepted WSI patch. The sum of the attribution score across channels is taken and then the absolute value is taken. A heatmap of the top 25% pixels that contribute to the second ML model's decision may be overlaid over a grayscale version of the (original) WSI to show to the system user which regions contributed the most to the decision.

EHR Data Processing

EHR data for a subject (individual/patient) in the context of this disclosure may be referred to as "medical data relating to a subject, including information relating to a diagnosis of breast cancer in the subject".

EHR generally includes clinical data, history, lab tests, demographics and other patient clinical information. Preprocessing may include removing any feature which is absent from above a threshold amount of the training data records/instances (e.g. removing features with more than 75% missing values). Uninformative features like identifiers and constant fields from EHRs are filtered out. Features remaining are categorized into numerical (e.g. diagnosis age), ordinal (e.g. IHC scores) or categorical (e.g. ICD-10 classification). Categorical and ordinal values are converted into numerical values. The preprocessing further includes one-hot encoding the categorical values and applying z-scoring (based on the mean and standard deviation of the training data used to train the third ML model) according to:

$$z = (x - \mu) / \sigma$$

where x is the input, $\mu$ is the mean and $\sigma$ is the standard deviation.

Examples of features that may be included in the medical data relating to a subject is shown below, together with descriptions some example values:

Diagnosis Age
an age of the subject when the subject was first diagnosed with breast cancer 62.0, 63.0, 61.0, 54.0, 50.0

Days to Sample Collection
a length of time between initial diagnosis of the subject with breast cancer and the tissue sample collection 57.0, 63.0, 394.0, 637.0, 162.0

Disease Free (Months)
a length of time the subject was disease free between the diagnosis of breast cancer and a previous diagnosis of breast cancer
0.33, 0.0, 13.01, 12.32, 12.65

Fraction Genome Altered
a Fraction of Genome Altered, FGA, measurement
0.0, 0.0001, 0.0002, 0.1153, 0.0004

Positive Finding Lymph Node Hematoxylin and Eosin Staining Microscopy Count
a Positive Lymph Node count based on Hematoxylin and Eosin Staining Microscopy 0.0, 1.0, 2.0, 3.0, 4.0

Positive Finding Lymph Node Keratin Immunohistochemistry Staining Method Count a Positive Lymph Node count based on a Keratin Immunohistochemistry Staining Method
0.0, 1.0, 2.0, 3.0, 4.0

Lymph Node(s) Examined Number
a number of Lymph Nodes Examined
2.0, 1.0, 3.0, 4.0, 10.0

Mutation Count
a Mutation Count
24.0, 22.0, 19.0, 23.0, 21.0

Sample Initial Weight
a tissue sample weight (of a tissue collected for breast cancer diagnosis/analysis) 110.0, 120.0, 100.0, 130.0, 200.0

TMB (nonsynonymous)
a nonsynonymous Tumor Mutational Burden, TMB
0.633333333, 0.766666667, 0.7, 0.6, 0.833333333

ER Status IHC Percent Positive
an estrogen receptor positive rate
90-99%, <10%, 70-79%, 80-89%, 10-19%

HER2 ihc score
an HER2 IHC test result
1.0, 2.0, 3.0, 0.0

PR status ihc percent positive
a progesterone receptor positive rate
<10%, 90-99%, 70-79%, 10-19%, 80-89%

American Joint Committee on Cancer Metastasis Stage Code
an American Joint Committee on Cancer, AJCC, Metastasis Stage Code
M0, MX, M1, cM0 (i+)

Neoplasm Disease Lymph Node Stage American Joint Committee on Cancer Code
an AJCC Neoplasm Disease Lymph Node Stage Code
N0, N1a, N0 (i-), N1, N2a

Neoplasm Disease Stage American Joint Committee on Cancer Code
an AJCC Neoplasm Disease Stage Code
Stage IIA, Stage IIB, Stage IIIA, Stage I, Stage IA

American Joint Committee on Cancer Publication Version Type
The version or edition of the American Joint Committee on Cancer Cancer Staging Handbooks, a publication by the group formed for the purpose of developing a system of clinical staging for cancer that is acceptable to the American medical profession and is compatible with other accepted classifications
6th, 7th, 5th, 4th, 3rd

American Joint Committee on Cancer Tumor Stage Code
an AJCC Tumor Stage Code
T2, T1c, T3, T1, T4b

Cancer Type Detailed
a breast Cancer Type
Breast Invasive Ductal Carcinoma, Breast Invasive Lobular Carcinoma, Breast Mixed Ductal and Lobular Carcinoma, Breast Invasive Mixed Mucinous Carcinoma, Metaplastic Breast Cancer

Disease Free Status
a Disease Free Status of the subject
0:DiseaseFree, 1:Recurred/Progressed

ER Status By IHC
a breast carcinoma estrogen receptor Status
Positive, Negative, Indeterminate

Ethnicity Category an Ethnicity of the subject
NOT HISPANIC OR LATINO, HISPANIC OR LATINO

HER2 fish status
an HER2 FISH test result
Negative, Positive, Equivocal, Indeterminate, [Not Evaluated]

Neoplasm Histologic Type Name
a Neoplasm Histologic Type Name
Infiltrating Ductal Carcinoma, Infiltrating Lobular Carcinoma, Other, Mixed Histology, Mucinous Carcinoma

Neoadjuvant Therapy Type Administered Prior To Resection Text an indication of whether the subject has received Neoadjuvant Therapy No, Yes

Prior Cancer Diagnosis Occurrence
an indication of whether the subject has been previously diagnosed with cancer No, Yes

ICD-10 Classification
an ICD-10 Classification
C50.9, C50.3, C50.4, C50.2, C50.8

International Classification of Diseases for Oncology, Third Edition ICD-O-3 Histology Code
an ICD-O-3 Histology Code
8500/3, 8520/3, 8522/3, 8523/3, 8480/3

International Classification of Diseases for Oncology, Third Edition ICD-O-3 Site Code an ICD-O-3 Site Code
C50.9, C50.3, C50.4, C50.2, C50.8

IHC-HER2
an HER2 IHC test result (presented in a different format from the information "HER2 ihc score" noted above)
Negative, Equivocal, Positive, Indeterminate

Primary Lymph Node Presentation Assessment Ind-3
an indication of whether a lymph node assessment was performed
YES, NO

Menopause Status
a Menopause Status
Post (prior bilateral ovariectomy OR >12 mo since LMP with no prior hysterectomy), Pre (<6 months since LMP AND no prior bilateral ovariectomy AND not on estrogen replacement), Peri (6-12 months since last menstrual period), Indeterminate (neither Pre or Postmenopausal)

Metastatic tumor indicator
a Metastatic tumor indicator
NO, YES

First Pathologic Diagnosis Biospecimen Acquisition Method Type
a First Pathologic Diagnosis Biospecimen Acquisition Method Type Core needle biopsy, Tumor resection, Fine needle aspiration biopsy, Other method, specify:, Excisional Biopsy

Micromet detection by ihc
a micrometastasis indicator
NO, YES

OCT embedded
whether the optimal cutting temperature (OCT) process was used before mounting slices (sections) of the tissue sample onto slides for analysis
True, False

Oncotree Code
an Oncotree Code
IDC, ILC, MDLC, IMMC, MBC

Disease Surgical Margin Status
a Surgical Margin Status
Negative, Positive, Close

Patient Primary Tumor Site
a Primary Tumor Site
Left Upper Outer Quadrant, Left, Right Upper Outer Quadrant, Right, Left Upper Inner Quadrant

Tissue Prospective Collection Indicator
Text indicator for the time frame of tissue procurement, indicating that the tissue was procured in parallel to the study or before (this information may be included only in the training data which is based on the "study")
NO, YES

PR status by ihc
Breast carcinoma progesterone receptor status
Positive, Negative, Indeterminate

Race Category
race information of the subject
WHITE, BLACK OR AFRICAN AMERICAN, ASIAN, AMERICAN INDIAN OR ALASKA NATIVE

Tissue Retrospective Collection Indicator
Text indicator for the time frame of tissue procurement, indicating that the tissue was obtained and stored prior to the initiation of the study (this information may be included only in the training data which is based on the "study")
YES, NO

Staging System
The process (staging system) used in determining the extent to which the breast cancer has grown and spread
Axillary lymph node dissection alone, Sentinel node biopsy alone, Sentinel lymph node biopsy plus axillary dissection,
No axillary staging, Other

Surgical procedure first
a breast carcinoma surgical procedure
Modified Radical Mastectomy, Other, Lumpectomy, Simple Mastectomy

Tissue Source Site
a Tissue Source Site
BH, A2, E2, A8, D8

Person Neoplasm Status
a Neoplasm Status
TUMOR FREE, WITH TUMOR

survival (months)
a length of time the subject survived since the diagnosis of breast cancer 0, 1, 6, 12

The third ML model may comprise a neural network. In an implementation example, the model architecture may comprise a classification head on top of an MLP backbone. The MLP network may be applied to the numerical data directly. The backbone produces an intermediate representation an the classification head produces a modality-specific prediction (output logits).

In implementation example, the numerical data is converted into textual key-value pairs, where the key is the feature name and the value is the corresponding feature value. in this example the third ML model comprises a classification head on top of a Transformer encoder. The Transformer produces the intermediate representation and the classification head on top of the intermediate representation produces the clinical modality prediction (output logits). For example, for the Transformer encoder, a pretrained BERT and RoBERTa (J. Devlin, et al, Bert: Pre-training of deep bidirectional transformers for language understanding, arXiv preprint arXiv:1810.04805 (2018) and Y. Liu, et al, Roberta: A robustly optimized bert pretraining approach, arXiv preprint arXiv: 1907.11692 (2019)) may be used after fine-tuning.

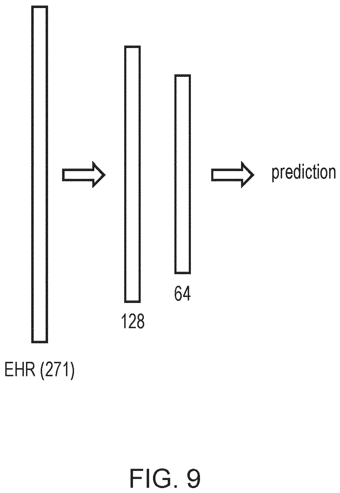

In a specific implementation example, the third ML model comprises an MLP with 128 and 64 channel hidden layers, ReLU activation and instance normalization, illustrated in Figure 9.

Explainability may be provided for the clinical features using Integrated Gradients. IG may be used to calculate the attribution of each feature in the input. Since one-hot encoding contributes more than one feature for each raw categorical feature, the contribution for all of the one-hot vector is summed and assigned to the raw feature. The absolute value is taken and the features are ranked according to their contribution to the prediction. This presents to the system user which features were more important for arriving at the third ML model's decision.

Fusion Processing

The fusion processing comprises integrating the single modality model outputs (logits) into a multimodal prediction. The fusion processing uses the output logits from the single-modality first to third ML models. For each modality and for each class output, the fusion processing uses a learnable weight parameter. The modality-specific outputs are scaled using these weights and summed to produce multi-modal weighted logits prediction. This is normalized into the multimodal probability output by applying a Softmax function.

If the output logits of model i (first to third ML models i = 1, 2, 3) are $Lg^{(i)} \in R^d$ where d is the output dimension, then the weighted logits output is defined as

$$Lg_j = (1/M) * \sum(\alpha_{ij} * Lg_j^{(i)}), \text{ sum is from } i = 1 \text{ to } M.$$

where $Lg_j^{(i)}$ is the jth element of the output of the ith modality (i.e. the logit for class/subtype j and modality i), $Lg_j$ is the jth element of the multimodal prediction logits, M is the number of modalities and $\alpha_{ij}$ are the learnable/trainable weights. The multimodal logits are then normalized into a probability distribution using a Softmax function. When classifying a subject's/patient's breast cancer as a subtype, the probability distribution is used to determine the classification. For example, the most likely subtype is output as the classification.

As mentioned above, the subject may have their data in different locations. This may be a CNV done in a certain lab, clinical history and EHR kept in a certain hospital or WSI stored in yet another lab. The data do not need to be moved into a central data storage for making a multimodal prediction. Each facility can use the single-modality ML model concerned on its own local data stored on premises or private storage. The single modality ML model can process the data locally and produce a single-modality prediction and explainability. Then the model outputs are serialized and communicated so that they can be used in the fusion processing. The fusion processing then can use only the model outputs (logits), without any information about the data used to produce these outputs, to produce the multi-modal prediction.

Training

As mentioned above, the training is caried out in two phases - in the first phase the first to third ML models are trained (in what may be referred to as first to third ML model training processes, respectively), and in the second phase the weights used in the fusion processing are trained (in what may be referred to as a fourth training process) with the first to third ML models' weights frozen.

In general, each of the first to third ML model training processes comprises using training data (CNV/WSI/medical data) relating to a training subject to predict a subtype for the training subject from among the plurality of breast cancer subtypes, comparing the predicted subtype with ground truth data indicating a ground truth subtype or the training subject, and adjusting at least one weight of the ML model concerned based on the comparison. Training of each ML model and the weights for the fusion processing is described in more detail below.

For the training, in an implementation example, CNV, WSI and EHR data obtained from the TCGA-BRCA dataset is used (WSIs are obtained directly from TCGA (cancer.gov/ccg/research/genome-sequencing/tcga), while CNV and EHR are obtained from cBioPortal (cbioportal.org/)), and the PAM50 breast cancer classification is obtained from an independent publication (Netanely, D., et. al. (2016), Expression and methylation patterns partition luminal-A breast tumors into distinct prognostic subgroups, Breast Cancer Research, 18, 1-16) - the PAM50 classifications in the publication were obtained using an RNA analysis and are therefore considered ground truth labels. Instances which do not have all three data modalities or with no PAM50 classification are filtered out. In this training implementation, the number of cases after filtration is 977.

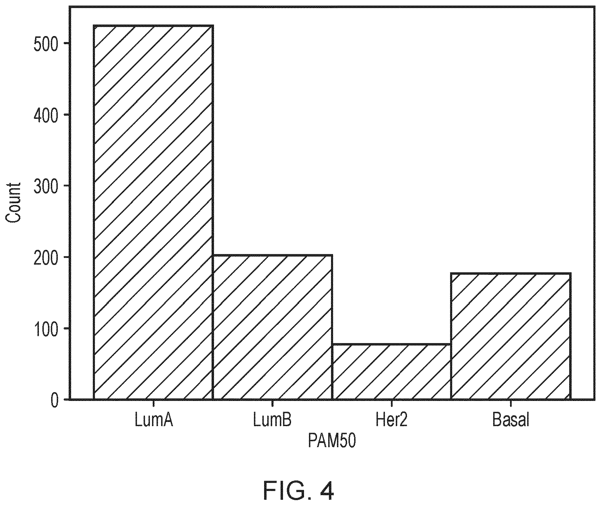

PAM50 classifies each case into one of four (sometimes five as mentioned above, but here "normal-like" is omitted) breast cancer subtypes: Luminal A, Luminal B, Her2-enriched and Basal-like. Figure 4 illustrates the sub-types of the 977 instances in the training data. Based on the PAM50 subtyping, the dataset is highly imbalanced as shown in Figure 4. Hence to obtain a better classification performance a balancing strategy is used. Two balancing strategies are used: oversampling and loss weighting. In oversampling, each class (sub-type) is resampled to have the same size as the majority class by repeating samples. In loss weighting, the individual class losses are rescaled inversely proportionally to the frequency of the class in the dataset. The dataset is divided case-wise into 80% training, 10% validation and 10% testing. Training of each ML model and the weights for the fusion processing, in the context of the above-mentioned implementation example using the training data mentioned above is described in more detail below

First ML Model Training

The CNV data is preprocessed by removing genes with missing values. This leaves around 23k genes for further processing. The preprocessing includes z-scoring the CNV values using the mean and standard deviation of the training data according to:

$$z = (x - \mu) / \sigma$$

where x is the input, $\mu$ is the mean and $\sigma$ is the standard deviation.

The first ML model's weights are initialized by setting bias to zero and initializing other weights according to:

$$w \sim N(0, \sigma)$$

$$\sigma = 1 / \sqrt{(H_{in})}$$

where $H_{in}$ is the number of input features, where w indicates the weights, and N $(0, \sigma)$ is the normal distribution. The first ML model is trained for 200 epochs using a stochastic optimization, e.g. Adam optimizer (Diederik P. Kingma and Jimmy Ba, Adam: A Method for Stochastic Optimization, 2017, arxiv.org/abs/1412.6980), and a dropout of 0.5.

Second ML Model Training

The training WSI data is preprocessed as described above for WSI data more generally. The pixel values are rescaled to [0.0, 1.0] (i.e. rescaled to be between 0 and 1) and normalized using mean =[0.485, 0.456, 0.406] and std= [0.229, 0.224, 0.225].

Training the second ML model follows weak supervision. Since the ground truth labels (classifications) are at the subject-level, the individual patches are assigned the case label; the second ML model is trained patch-wise. However at inference the model is evaluated by pooling the output to produce a single prediction per case/subject.

When doing the pooling at the intermediate representation level, two strategies (among others) may be considered. The first strategy uses two-phases. In the first phase, the model is trained patch-wise. Then the backbone is frozen and uses to encode and pool intermediate representations. Then a new classifier is used to classify the pooled intermediate representations. In the other strategy, the model is trained with pooling from the beginning by taking the mean of sampled patches for each subject. At inference time, the mean of all of the patches for each patient is used. When doing aggregation at the intermediate representation level, in order to make the process more computationally tractable, only the top 50 patches for each subject may be used, ranked based on the area of the segmented tissue content.

For Inceptionv3, VGG16 and Dinov2, the input patches are further pre-processed by: 1) resizing into 342×342 for Inceptionv3 or 256×256 for VGG16 and Dinov2 using bilinear interpolation, 2) applying a central crop of 299×299 for Inceptionv3 or 224×224 for VGG16 and Dinov2 and 3) rescaling the pixel values to [0.0, 1.0] and normalizing using mean= [0.485, 0.456, 0.406] and std=[0.229, 0.224, 0.225].

In an alternative implementation example for the second ML model training weights pretrained on ImageNet (Deng, J. et al (2009) Imagenet: A large-scale hierarchical image database, In 2009 IEEE conference on computer vision and pattern recognition (pp. 248-255), Ieee) may be used in the second ML model (but the fully-connected layer removed).

The second ML model is trained (e.g. for 200 epochs) using a stochastic optimization, e.g. Adam optimizer.

Third ML Model Training

The training data is preprocessed as described above for EHR data more generally. Furthermore in the training phase missing values are imputed using a k-Nearest Neighbours algorithm.

The number of input features in the training EHR data after expansion done by the one-hot encoding on the training data according to the implementation example mentioned above is 271. The third ML model is trained (e.g. for 200 epochs) using a stochastic optimization, e.g. Adam optimizer.

Training the Weights for Fusion Processing

After the modality specific models are trained, the integration module is trained to do the classification using only the outputs from the other models. That is, the first to third ML models are applied on training data (each model applied on its specific data-type) relating to a training subject and the output logits are fed to the fusion processing. The weights are used to generate a weighted sum and thus a classification, which is compared with the ground truth data indicating a corresponding ground truth classification for the training subject. Based on the comparison, the weights are adjusted. The training is implemented (e.g. for 200 epochs) using a stochastic optimization, e.g. Adam optimizer.

Training schemes other than stochastic optimization (stochastic gradient descent - SGD) may be used, for example including Simulated Annealing and Evolutionary Strategies. For deep learning, however, SGD is the most efficient.

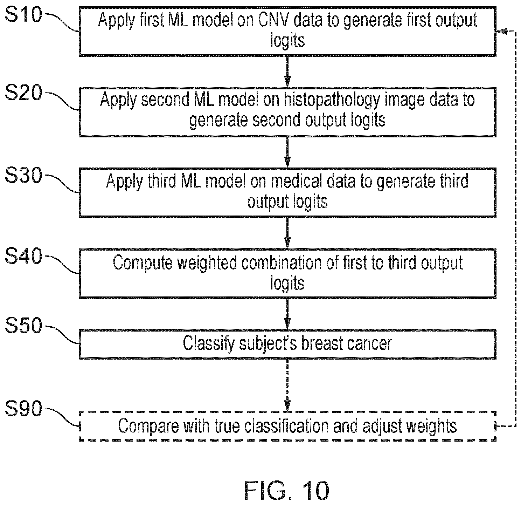

Figure 10 is a diagram illustrating a method comprising steps S10-S50, and optionally step S90.

Step S10 comprises applying a first ML model on CNV data to generate first output logits. That is, step S10 comprises applying a first ML model on CNV data relating to a subject to generate first output logits corresponding respectively to a plurality of breast cancer sub-types, wherein the first ML model has been trained to classify CNV data as corresponding to a sub-type of the plurality of breast cancer sub-types.

Step S20 comprises applying a second ML model on histopathology image data to generate second output logits. That is, step S10 comprises applying a second ML model on histopathology image data relating to the subject to generate second output logits corresponding respectively to the plurality of breast cancer sub-types, wherein the second ML model has been trained to classify histopathology data as corresponding to a sub-type of the plurality of breast cancer sub-types.

Step S30 comprises applying a third ML model on medical data to generate third output logits. That is, step S30 comprises applying a third ML model on medical data relating to the subject, including information relating to a diagnosis of breast cancer in the subject, to generate third output logits corresponding respectively to the plurality of breast cancer sub-types, wherein the third ML model has been trained to classify medical data, including information relating to a diagnosis of breast cancer, as corresponding to a sub-type of the plurality of breast cancer sub-types.

Step S40 comprises computing a weighted combination of the first to third output logits. That is, Step S40 comprises computing a weighted combination of the first output logits, the second output logits, and the third output logits.

Step S60 comprises classifying the subject's breast cancer. That is, step S50 comprises based on the weighted combination, classifying the subject's breast cancer as a sub-type of the plurality of breast cancer sub-types.

The subject may be referred to as a patient or a target patient. The method may further comprise outputting a diagnosis comprising the classification.

Step S10 may be considered as corresponding to the function of the CNV module 10 and/or the steps S12-S16, and description applying to the CNV module 10 and the steps S12-S16 is in some implementations applicable to the step S10 and vice versa. Step S20 may be considered as corresponding to the function of the WSI module 20 and/or the steps S22-S28, and description applying to the WSI module 20 and the steps S22-S28 is in some implementations applicable to the step S20 and vice versa. Step S30 may be considered as corresponding to the function of the EHR module 30 and/or the steps S32-S36, and description applying to the EHR module 30 and the steps S32-S36 is in some implementations applicable to the step S30 and vice versa. Steps S40-S50 may be considered as corresponding to the function of the integration module 60 and/or the step S60, and description applying to the integration module 60 and the step S60 is in some implementations applicable to the step S10 and vice versa. In particular, the first to third ML models in the Figure 10 method may be the first to third ML models described with reference to the modules 10, 20, 30. Further in particular, the histopathology image data may comprise WSI image data and/or the medical data may comprise EHR data. Furthermore, in particular, the weights used in step S40 are learned/trained. Further, in particular the breast cancer sub-types are the PAM50 sub-types.

Step S90 is optional and comprises comparing the classification output in step S50 with a true classification (i.e. a ground truth classification from training data) and adjusting the weights used in step S40. A training method (may be referred to as a fourth training process) for training the step S40 weights comprises the step S90, and as shown the training method returns to step S10, and in this training method the CNV data, histopathology image data, and medical data are training data relating to a training subject. The classification and adjustment is repeated/iterated and thus carried out for training data relating to a plurality of training subjects. Also disclosed herein are training processes for training the first to third ML models. In the Figure 10 methods the first to third ML models are already trained (and their weights frozen).

In an implementation example, the Figure 10 method is implemented using a plurality of processors or computing devices, in particular using a first at least one processor or computing device for applying the second ML model, the first at least one processor or computing device being comparatively larger and/or having a higher processing capacity than a second at least one processor or computing device used for any of: applying the first ML model; applying the third ML model; computing the weighted combination; and classifying the subject's breast cancer. This is advantageous because applying the second ML model may be considered more computationally expensive than the other method steps. Furthermore, at least one graphical processing unit (GPU) may be used for applying the second ML model whilst a least one central computing unit (CPU) may be used for at least one other method step. It will be appreciated that a similar division of processing load may be applied to the system 100 previously described. Furthermore, this division of processing load may apply when training the ML models and weights and/or when classifying a target patient's breast cancer after training has been completed.

In line with Figure 10, for example, and according to a first embodiment there is disclosed herein a computer-implemented method comprising: applying a first machine learning, ML, model on copy number variation, CNV, data relating to a subject to generate first output logits corresponding respectively to a plurality of breast cancer sub-types, wherein the first ML model has been trained to classify (training) CNV data as corresponding to a sub-type of the plurality of breast cancer sub-types; applying a second ML model on histopathology image data relating to the subject to generate second output logits corresponding respectively to the plurality of breast cancer sub-types, wherein the second ML model has been trained to classify (training) histopathology data as corresponding to a sub-type of the plurality of breast cancer sub-types; applying a third ML model on medical data relating to the subject, including information relating to a diagnosis of breast cancer in the subject, to generate third output logits corresponding respectively to the plurality of breast cancer sub-types, wherein the third ML model has been trained to classify (training) medical data, including information relating to a diagnosis of breast cancer (relating to a training subject(s)), as corresponding to a sub-type of the plurality of breast cancer sub-types; computing a weighted combination of the first output logits, the second output logits, and the third output logits; and based on the weighted combination, classifying the subject's breast cancer as a sub-type of the plurality of breast cancer sub-types.

The first output logits may be indicative of a probability of the CNV data corresponding to each of the plurality of breast cancer sub-types, respectively, and the second output logits may be indicative of a probability of the histopathology image data corresponding to each of the plurality of breast cancer sub-types, respectively, and the third output logits may be indicative of a probability of the medical data corresponding to each of the plurality of breast cancer sub-types, respectively.

The first ML model may comprise a neural network.

The first ML model may comprise a feedforward network.

The first ML model may comprise a feedforward network comprising a scaled exponential linear unit (SELU) activation function and/or using alpha-dropout.

The first ML model may comprise a self-normalizing network (SNN).

Applying the first ML model on the CNV data relating to the subject may comprise normalizing the CNV data using z-score normalization (based on training CNV data).

The histopathology image data relating to the subject may comprise a whole scale image, WSI.

Applying the second ML model on the histopathology image data relating to the subject may comprise dividing the WSI into a plurality of patches, filtering a saturation channel of the WSI using median blurring, thresholding the WSI according to the filtered saturation channel to generate a mask, and selecting patches for input to the second ML model based on tissue content using the mask.

Selecting patches for input to the second ML model based on tissue content using the mask may comprise selecting patches that have a (percentage) tissue content above a tissue content threshold according to the mask.

Applying the second ML model on the histopathology image data relating to the subject may comprise downsampling the selected patches and/or normalizing pixel values of the selected patches.

The second ML model may comprise a neural network.

The second ML model may comprise a fully-connected neural network.

The second ML model may comprise a convolutional neural network.

The medical data relating to the subject may comprise any of: an age of the subject when the subject was first diagnosed with breast cancer; a length of time between initial diagnosis of the subject with breast cancer and the tissue sample collection; a length of time the subject was disease free between the diagnosis of breast cancer and a previous diagnosis of breast cancer; a Fraction of Genome Altered, FGA, measurement; a Positive Lymph Node count based on Hematoxylin and Eosin Staining Microscopy; a Positive Lymph Node count based on a Keratin Immunohistochemistry Staining Method; a number of Lymph Nodes Examined; a Mutation Count; a tissue sample weight; a nonsynonymous Tumor Mutational Burden, TMB; an estrogen receptor positive rate; a progesterone receptor positive rate; an HER2 IHC test result; an American Joint Committee on Cancer, AJCC, Metastasis Stage Code; an AJCC Neoplasm Disease Lymph Node Stage Code; an AJCC Neoplasm Disease Stage Code; an AJCC Tumor Stage Code; an AJCC Publication Version Type; a breast Cancer Type; a Disease Free Status of the subject; a breast carcinoma estrogen receptor Status; a breast carcinoma progesterone receptor Status; an Ethnicity of the subject; an HER2 FISH test result; a Neoplasm Histologic Type Name; an indication of whether the subject has received Neoadjuvant Therapy; an indication of whether the subject has been previously diagnosed with cancer; an ICD-10 Classification; an ICD-O-3 Histology Code; an ICD-O-3 Site Code; an indication of whether a lymph node assessment was performed; a Menopause Status; a Metastatic tumor indicator; a First Pathologic Diagnosis Biospecimen Acquisition Method Type; a micrometastasis indicator; an Oncotree Code; an indication of whether an optimal cutting temperature (OCT) process was used for the tissue sample; a Surgical Margin Status; a Primary Tumor Site; race information of the subject; a breast carcinoma surgical procedure; a Tissue Source Site; a Neoplasm Status; and a length of time the subject survived since the diagnosis of breast cancer.

The breast cancer type may comprise an indication of a type of breast cancer from among a plurality of types including Breast Invasive Ductal Carcinoma, Breast Invasive Lobular Carcinoma, Breast Mixed Ductal and Lobular Carcinoma, Breast Invasive Mixed Mucinous Carcinoma, and Metaplastic Breast Cancer.

The Neoplasm Histologic Type Name may comprise an indication of a type name from among a plurality of Neoplasm Histologic Type Names including Infiltrating Ductal Carcinoma, Infiltrating Lobular Carcinoma, Mucinous Carcinoma.

The First Pathologic Diagnosis Biospecimen Acquisition Method Type may comprise an indication of a procedure used for the diagnosis of breast cancer from among a plurality of procedures including Core needle biopsy, Tumor resection, Fine needle aspiration biopsy, and Excisional Biopsy.

The medical data relating to the subject may comprise any of: an age of the subject when the subject was first diagnosed with breast cancer; a Fraction of Genome

Altered, FGA, measurement; a Positive Lymph Node count based on Hematoxylin and Eosin Staining Microscopy; a Positive Lymph Node count based on a Keratin Immunohistochemistry Staining Method; a nonsynonymous Tumor Mutational Burden, TMB; an estrogen receptor positive rate; a progesterone receptor positive rate; an HER2 IHC test result; an American Joint Committee on Cancer, AJCC, Metastasis Stage Code; an AJCC Neoplasm Disease Lymph Node Stage Code; an AJCC Neoplasm Disease Stage Code; an AJCC Tumor Stage Code; a breast Cancer Type; a Disease Free Status of the subject; a breast carcinoma estrogen receptor Status; a breast carcinoma progesterone receptor Status; an Ethnicity of the subject; an HER2 FISH test result; a Neoplasm Histologic Type Name; an indication of whether the subject has received Neoadjuvant Therapy; an indication of whether the subject has been previously diagnosed with cancer; an ICD-10 Classification; an ICD-O-3 Histology Code; an ICD-O-3 Site Code; a Menopause Status; an Oncotree Code; a Primary tumor Site; and race information of the subject.

The medical data relating to the subject may comprise any of: an age of the subject when the subject was first diagnosed with breast cancer; an estrogen receptor positive rate; a progesterone receptor positive rate; an HER2 IHC test result; an American Joint Committee on Cancer, AJCC, Metastasis Stage Code; an AJCC Neoplasm Disease Lymph Node Stage Code; an AJCC Neoplasm Disease Stage Code; an AJCC Tumor Stage Code; a breast Cancer Type; an Ethnicity of the subject; an HER2 FISH test result; a Neoplasm Histologic Type Name; an ICD-10 Classification; an ICD-O-3 Histology Code; an ICD-O-3 Site Code; a Menopause Status; an Oncotree Code; a Primary Tumor Site; and race information of the subject.

The medical data relating to the subject may comprise electronic health record, EHR, data.

Applying the third ML model on the medical data relating to the subject may comprise converting ordinal and categorical values in the medical data into numerical values (before input to the third ML model).

Applying the third ML model on the medical data relating to the subject may comprise imputing missing values in the medical data relating to the subject using a nearest neighbors algorithm based on medical data relating to a plurality of other subjects (before input to the third ML model).

Applying the third ML model on the medical data relating to the subject may comprise one-hot encoding the values in the medical data and normalizing the encoded values using z-score normalization (before input to the third ML model).

The third ML model may comprise a neural network.

The computer-implemented method may comprise obtaining the CNV data relating to the subject and/or the histopathology image data relating to the subject and/or the medical data relating to the subject.

The first ML model may have been trained according to a first ML model training process comprising: applying the first ML model on training CNV data relating to a plurality of training subjects to predict a sub-type from among the plurality of breast cancer sub-types for each training subject; comparing the predicted sub-types to ground truth data indicating ground truth sub-types corresponding respectively to the training subjects; adjusting at least one weight of the first ML model based on the comparison.

The first ML model training process may comprise repeatedly applying the first ML model on training CNV data to predict sub-types for test subjects, comparing the predicted sub-types with ground-truth sub-types, and adjusting at least one weight of the first ML model based on the comparison for a threshold number of iterations or until a loss threshold is met.

The computer-implemented method may comprise, before applying the first ML model on the CNV data relating to the subject, performing the first ML model training process to train the first ML model to classify CNV data as corresponding to a sub-type of the plurality of breast cancer sub-types.

The second ML model may have been trained according to a second ML model training process comprising: applying the second ML model on training histopathology image data relating to a plurality of training subjects to predict a sub-type from among the plurality of breast cancer sub-types for each training subject; comparing the predicted sub-types to ground truth data indicating ground truth sub-types corresponding respectively to the training subjects; adjusting at least one weight of the second ML model based on the comparison.

The second ML model training process may comprise repeatedly applying the second ML model on training histopathology image data to predict sub-types for test subjects, comparing the predicted sub-types with ground-truth sub-types, and adjusting at least one weight of the second ML model based on the comparison for a threshold number of iterations or until a loss threshold is met.

The second ML model training process may comprise training the second ML model patch-wise.

Predicting a sub-type from among the plurality of breast cancer sub-types for each training subject may comprise, for each training subject, predicting a sub-type for each patch, and wherein comparing the predicted sub-types to the ground truth data comprises performing the comparison for each patch.

The computer-implemented method may comprise, before applying the second ML model on the histopathology image data relating to the subject, performing the second ML model training process to train the second ML model to classify histopathology image data as corresponding to a sub-type of the plurality of breast cancer sub-types.

The third ML model may have been trained according to a third ML model training process comprising: applying the third ML model on training medical data, including information relating to a diagnosis of breast cancer, relating to a plurality of training subjects to predict a sub-type from among the plurality of breast cancer sub-types for each training subject; comparing the predicted sub-types to ground truth data indicating ground truth sub-types corresponding respectively to the training subjects; adjusting at least one weight of the third ML model based on the comparison.

The third ML model training process may comprise repeatedly applying the third ML model on training medical data, including information relating to a diagnosis of breast cancer, to predict sub-types for test subjects, comparing the predicted sub-types with ground-truth sub-types, and adjusting at least one weight of the third ML model based on the comparison for a threshold number of iterations or until a loss threshold is met. The computer-implemented method may comprise, before applying the third ML model on the medical data relating to the subject, performing the third ML model training process to train the third ML model to classify training medical data, including information relating to a diagnosis of breast cancer, as corresponding to a sub-type of the plurality of breast cancer sub-types.

In any of the first to third ML model training processes, comparing the predicted sub-types to the ground truth data may comprise computing a loss based on the comparison, and adjusting at least one weight of the ML model concerned comprises adjusting at least one weight based on the computed loss.

Classifying the subject's breast cancer as a sub-type of the plurality of breast cancer sub-types may comprise generating, based on the weighted combination, a probability distribution using a softmax function, and classifying the subject's breast cancer according to the probability distribution.

Computing the weighted combination of the first output logits, the second output logits, and the third output logits may comprise using first weights, second weights, and third weights corresponding respectively to the first, second, and third output logits, wherein the first weights, second weights, and third weights each comprise weights corresponding respectively to the plurality of breast cancer sub-types.

The first, second, and third weights may have been trained according to a (fourth) training process.

The (fourth) training process may comprise: computing a training weighted combination of first, second, and third training output logits, the first training output logits the result of applying the first ML model on training CNV data relating to a training subject, the second training output logits the result of applying the second ML model on training histopathology image data relating to the training subject, and the third training output logits the result of applying the third ML model on training medical data relating to the training subject, including information relating to a diagnosis of breast cancer in the training subject; based on the training weighted combination, classifying the training subject's breast cancer as a sub-type of the plurality of breast cancer sub-types; and comparing the classification with ground truth data indicating a ground-truth sub-type corresponding to the training subject and at least one of the first weights, the second weights, and the third weights based on the comparison.

The first, second, and third weights may have been trained by iterating the (fourth) training process for a plurality of training subjects for a threshold number of iterations or until a loss threshold is met.

The (fourth) training process may comprise repeatedly computing a training weighted combination of first, second, and third training output logits for a plurality of training subjects to classify the training subjects' breast cancer as a sub-type, comparing classification with ground truth sub-types, and adjusting at least one of the first, second, and third weights based on the comparison.

The computer-implemented method may comprise, before computing the weighted combination, training the first, second, and third weights according to the (fourth) training process.

The computer-implemented method may comprise performing the first, second, and third ML model training processes to train the first, second and third ML models before performing the (fourth) training process.

The subject may be a training subject and the method may comprise comparing the classification with ground truth data indicating a ground-truth sub-type corresponding to the training subject and adjusting at least one of the first weights, the second weights, and the third weights based on the comparison.

The subject may be a target patient and the computer-implemented method may comprise outputting a diagnosis of the target patient including the classifying of the subject's breast cancer as a sub-type of the plurality of breast cancer sub-types.

The plurality of breast cancer sub-types may comprise a plurality of PAM50 breast cancer sub-types.

The plurality of breast cancer sub-types may comprise Luminal A, Luminal B, Her2-enriched, and Basal-like.

The computer-implemented method may comprise using a first at least one processor or computing device for applying the second ML model, the first at least one processor or computing device being comparatively larger and/or having a higher processing capacity than a second at least one processor or computing device used for any of: applying the first ML model; applying the third ML model; computing the weighted combination; and classifying the subject's breast cancer.

The computer-implemented method may comprise using a first at least one processor or computing device for applying the second ML model and a second at least one processor or computing device for computing the weighted combination and classifying the subject's breast cancer, wherein the first at least one processor or computing device is larger and/or has a higher processing capacity than the second at least one processor or computing device.

The computer-implemented method may comprise using a plurality of processors and/or computing devices located in different/distinct (geographical) locations and/or in different medical facilities.

The computer-implemented method may comprise using a first at least one processor or computing device for applying the second ML model, using a second at least one processor or computing device for applying the first ML model, using a third at least one processor or computing device for applying the third ML model, and using a fourth at least one processor for computing the weighted combination, wherein the first, second, third, and fourth at least one processor or computing device are located in different/distinct (geographical) locations and/or in different medical facilities.

According to a second embodiment there is disclosed herein a computer program which, when run on a computer, causes the computer to carry out a method comprising: applying a first machine learning, ML, model on copy number variation, CNV, data relating to a subject to generate first output logits corresponding respectively to a plurality of breast cancer sub-types, wherein the first ML model has been trained to classify CNV data as corresponding to a sub-type of the plurality of breast cancer sub-types; applying a second ML model on histopathology image data relating to the subject to generate second output logits corresponding respectively to the plurality of breast cancer sub-types, wherein the second ML model has been trained to classify histopathology data as corresponding to a sub-type of the plurality of breast cancer sub-types; applying a third ML model on medical data relating to the subject, including information relating to a diagnosis of breast cancer in the subject, to generate third output logits corresponding respectively to the plurality of breast cancer sub-types, wherein the third ML model has been trained to classify medical data, including information relating to a diagnosis of breast cancer, as corresponding to a sub-type of the plurality of breast cancer sub-types; computing a weighted combination of the first output logits, the second output logits, and the third output logits; and based on the weighted combination, classifying the subject's breast cancer as a sub-type of the plurality of breast cancer sub-types.

According to a third embodiment there is disclosed herein an information processing apparatus comprising a memory and a processor connected to the memory, wherein the processor is configured to: apply a first machine learning, ML, model on copy number variation, CNV, data relating to a subject to generate first output logits corresponding respectively to a plurality of breast cancer sub-types, wherein the first ML model has been trained to classify CNV data as corresponding to a sub-type of the plurality of breast cancer sub-types; apply a second ML model on histopathology image data relating to the subject to generate second output logits corresponding respectively to the plurality of breast cancer sub-types, wherein the second ML model has been trained to classify histopathology data as corresponding to a sub-type of the plurality of breast cancer sub-types; apply a third ML model on medical data relating to the subject, including information relating to a diagnosis of breast cancer in the subject, to generate third output logits corresponding respectively to the plurality of breast cancer sub-types, wherein the third ML model has been trained to classify medical data, including information relating to a diagnosis of breast cancer, as corresponding to a sub-type of the plurality of breast cancer sub-types; compute a weighted combination of the first output logits, the second output logits, and the third output logits; and based on the weighted combination, classify the subject's breast cancer as a sub-type of the plurality of breast cancer sub-types.

According to a fourth embodiment there is disclosed herein a system comprising a plurality of computing devices configured to: apply a first machine learning, ML, model on copy number variation, CNV, data relating to a subject to generate first output logits corresponding respectively to a plurality of breast cancer sub-types, wherein the first ML model has been trained to classify CNV data as corresponding to a sub-type of the plurality of breast cancer sub-types; apply a second ML model on histopathology image data relating to the subject to generate second output logits corresponding respectively to the plurality of breast cancer sub-types, wherein the second ML model has been trained to classify histopathology data as corresponding to a sub-type of the plurality of breast cancer sub-types; apply a third ML model on medical data relating to the subject, including information relating to a diagnosis of breast cancer in the subject, to generate third output logits corresponding respectively to the plurality of breast cancer sub-types, wherein the third ML model has been trained to classify medical data, including information relating to a diagnosis of breast cancer, as corresponding to a sub-type of the plurality of breast cancer sub-types; compute a weighted combination of the first output logits, the second output logits, and the third output logits; and based on the weighted combination, classify the subject's breast cancer as a sub-type of the plurality of breast cancer sub-types.

The plurality of computing devices may comprise a first at least one processor or computing device configured to apply the second ML model, the first at least one processor or computing device being comparatively larger and/or having a higher processing capacity than a second at least one processor or computing device configured to any of: apply the first ML model; apply the third ML model; compute the weighted combination; and classify the subject's breast cancer.

The plurality of computing devices may comprise a first at least one processor or computing device configured to apply the second ML model and a second at least one processor or computing device configured to compute the weighted combination and classify the subject's breast cancer, wherein the first at least one processor or computing device is larger and/or has a higher processing capacity than the second at least one processor or computing device.

The plurality of computing devices may comprise a plurality of processors and/or computing devices located in different/distinct (geographical) locations and/or in different medical facilities.

The plurality of computing devices may comprise a first at least one processor or computing device configured to apply the second ML model, a second at least one processor or computing device configured to apply the first ML model, a third at least one processor or computing device configured to apply the third ML model, and a fourth at least one processor configured to compute the weighted combination, wherein the first, second, third, and fourth at least one processor or computing device are located in different/distinct (geographical) locations and/or in different medical facilities.

Worked Example

Described here is a worked example of the multi-modal prediction of PAM50 cancer subtype, specifically for the subject with sample id "tcga-a2-a0ev-01" from the dataset TCGA-BRCA.

The CNV input contains around 23k genes. This raw input is z-scored using the mean and standard deviation of the training dataset to give corresponding normalized gene values. A sample of eight CNV values together with their normalized CNV values are shown below.

"gene, CNV value, normalized (z-scored)":

MIR4694, 0, -1.501605
RAB2A, 0, -2.070566
RN7SL82P, 0, 0.435044
KLHL3, 1, 0.015845
CEP97, 0, -0.263007
MEX3B, 1, 0.161831
CCDC74B, 0, 0.266389
F8A1, 0, -1.594879

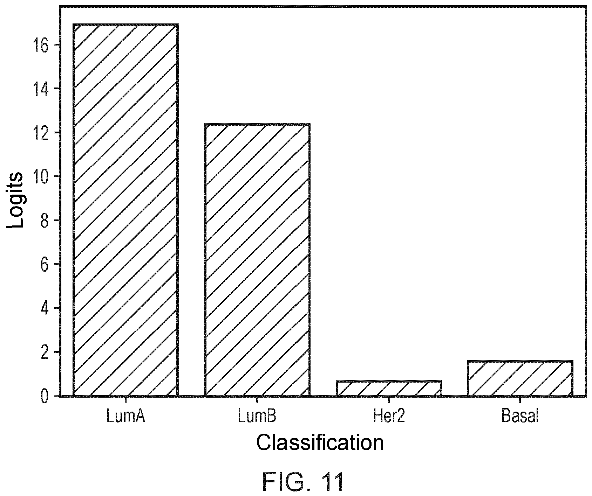

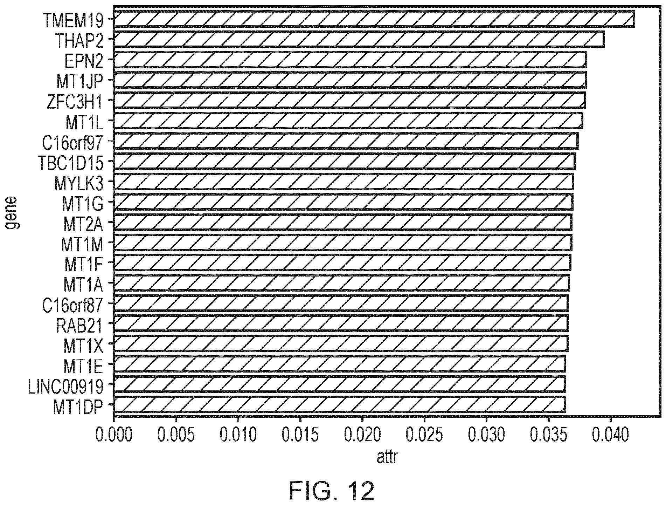

The first ML model (after training) is applied on the preprocessed CNV data for the subject and produces the output logits for this modality (first output logits). These first output logits are illustrated in Figure 11. For explainability, the first ML model uses Integrated gradients to produce the attribution for each input gene. The absolute values of these attribution weights are taken and the genes are ranked according to their contribution to the final output. The top 20 contributing genes for the subject concerned are illustrated in Figure 12.

The output logits are serialized and used for the multimodal prediction.

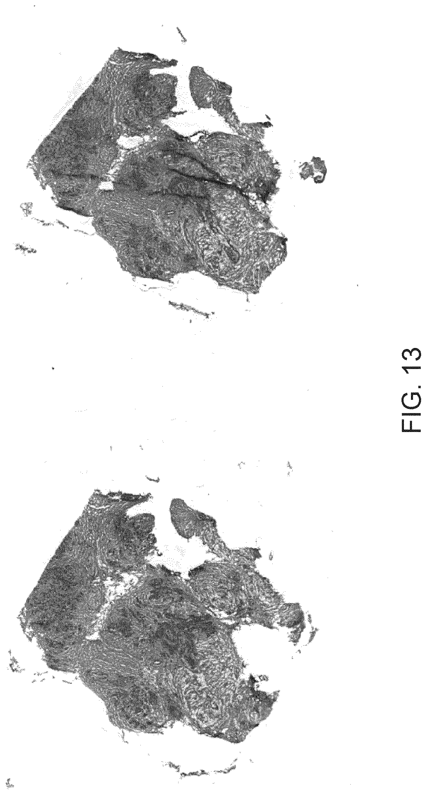

The raw input WSI for the subject "tcga-a2-a0ev-01" is the WSI image shown in Figure 13 (in black and white, but in practice the image may include colour).

A large area of the WSI is usually a background, so a segmentation algorithm is applied to separate the tissue from the background. In line with previous description, a mask is generated by:

- Extracting 256x256 patches from the WSI
- Converting the patches from RGB colour-space to HSV colour-space
- Applying median blurring to the saturation channel using a kernel size of 7
- Generating a binary mask by thresholding the saturation channel using a binary threshold of 20

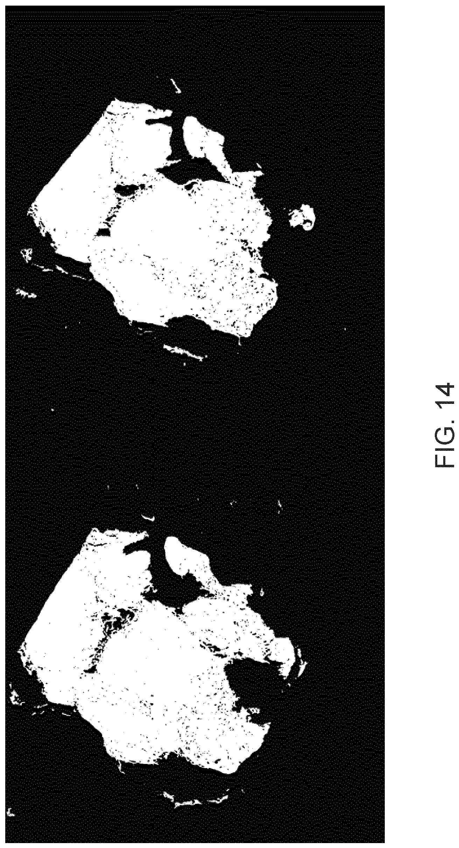

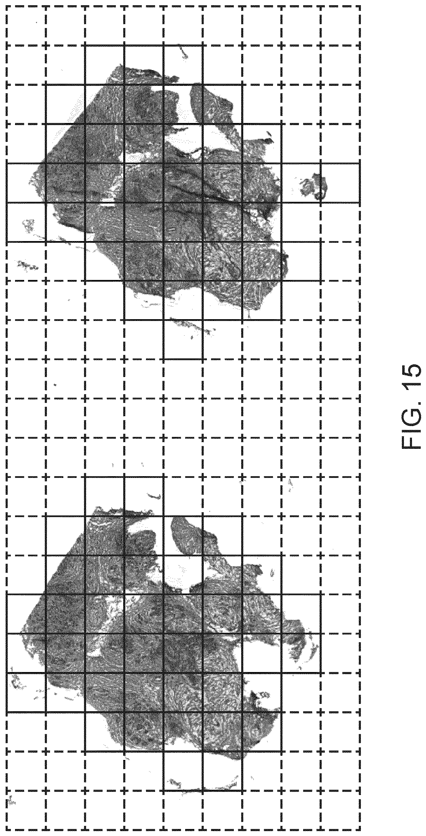

This generates a binary mask that differentiates the foreground from the background as shown Figure 14. Using this mask, the tissue content is calculated in each patch and patches that do not contain at least 5% tissue are filtered out. This process is visualized in Figure 15, in which solid lines indicate accepted patches that contain enough tissue, while broken/dashed lines indicate rejected patches that are mostly background.

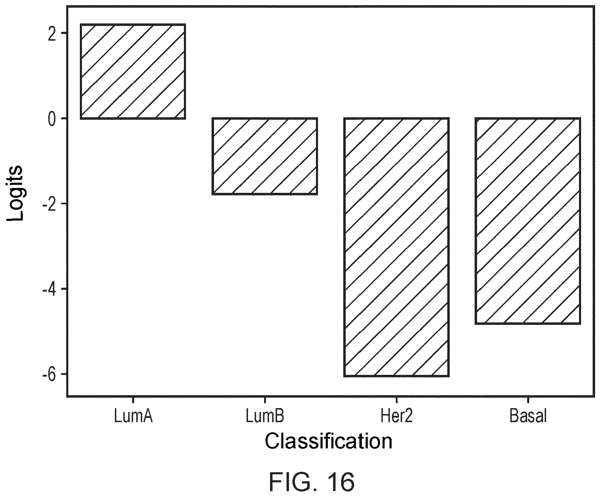

The accepted patches are 20x down-sampled and then transformed by resizing to 342x342 using bilinear interpolation and central cropping a 299x299 square. Pixel values are then rescaled to the range [0.0, 1.0] and normalized using mean=[0.485, 0.456, 0.406] and std=[0.229, 0.224, 0.225] from the training dataset. The accepted patches are then passed into the second ML model (after training). An output per patch is generated - these outputs are aggregated using the mean operator into an overall set of output logits (second output logits) for the subject. The second output logits for the subject are illustrated in Figure 16.

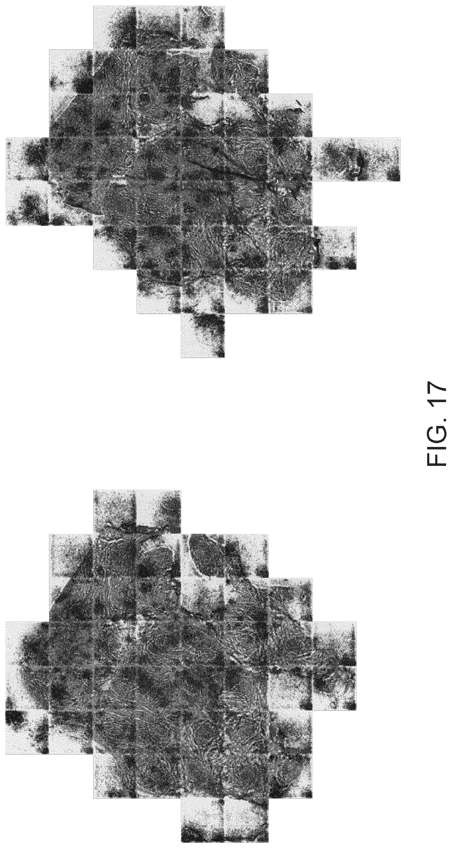

For explainability, the second ML model uses Integrated Gradients to calculate the attribution for each pixel in the WSI accepted patches. The sum of the attribution is taken across the image channels, and the absolute value of the attribution is computed. A heatmap of the top 25% contributing pixels overlayed over a grayscale version of the WSI is produced, as illustrated in Figure 17 (illustrated here in black and white, though colour may be used to indicate the heatmap).

The second output logits are serialized and used for the multimodal prediction.

The raw EHR data for the subject "tcga-a2-a0ev-01" is:

```
'{"Diagnosis Age":80.0,"Days to Sample Collection.":590.0,"Disease Free
(Months)":31.8,"Fraction Genome Altered":0.2086,"Positive Finding Lymph Node
Hematoxylin and Eosin Staining Microscopy Count":0.0,"Positive Finding Lymph
Node Keratin Immunohistochemistry Staining Method Count":0.0,"Lymph Node(s)
Examined Number":6.0,"Mutation Count":49.0,"Overall Survival
(Months)":31.8,"Sample Initial Weight":960.0,"TMB
(nonsynonymous)":1.766666667,"American Joint Committee on Cancer Metastasis
Stage Code":"M0","Neoplasm Disease Lymph Node Stage American Joint Committee
on Cancer Code":"N0 (i-)","Neoplasm Disease Stage American Joint Committee on
Cancer Code":"Stage IA","American Joint Committee on Cancer Publication
Version Type":"6th","American Joint Committee on Cancer Tumor Stage
Code":"T1c","Cancer Type Detailed":"Breast Invasive Ductal
Carcinoma","Disease Free Status":"0:DiseaseFree","ER Status By
IHC":"Positive","Ethnicity Category":"NOT HISPANIC OR LATINO","HER2 fish
status":"Negative","Neoplasm Histologic Type Name":"Infiltrating Ductal
Carcinoma","Neoadjuvant Therapy Type Administered Prior To Resection
Text":"No","Prior Cancer Diagnosis Occurence":"No","ICD-10
Classification":"C50.9","International Classification of Diseases for
Oncology, Third Edition ICD-0-3 Histology Code":"8500\\/3","International
```

Classification of Diseases for Oncology, Third Edition ICD-0-3 Site Code":"C50.9","IHC-HER2":"Negative","Primary Lymph Node Presentation Assessment Ind-3":null,"Menopause Status":"Post (prior bilateral ovariectomy OR >12 mo since LMP with no prior hysterectomy)","Metastatic tumor indicator":null,"First Pathologic Diagnosis Biospecimen Acquisition Method Type":"Core needle biopsy","Micromet detection by ihc":"YES","Oct embedded":true,"Oncotree Code":"IDC","Overall Survival Status":"0:LIVING","Disease Surgical Margin Status":"Negative","Patient Primary Tumor Site":"Left","Tissue Prospective Collection Indicator":"NO","PR status by ihc":"Positive","Race Category":"WHITE","Tissue Retrospective Collection Indicator":"YES","Staging System":"Sentinel node biopsy alone","Surgical procedure first":"Lumpectomy","Tissue Source Site":"A2","Person Neoplasm Status":"TUMOR FREE","Vial number":"A","ER Status IHC Percent Positive":"90-99%","HER2 ihc score":0.0,"PR status ihc percent positive":"90-99%"}'

This data is first pre-processed, including: ordinal and categorical values are converted into numerical values; missing values are imputed using k-NN; the categorical values are one-hot encoded; and the data is z-scored based on the statistics of the training dataset. Because of the one-hot encoding, this expands the input data for the subject into 271 features. A sample of the transformed preprocessed input data for the subject is:

'{"Diagnosis Age":1.6162747294,"Days to Sample Collection.":-0.3339608878,"Disease Free (Months)":-0.1537865172,"Fraction Genome Altered":-0.4727803659,"Positive Finding Lymph Node Hematoxylin and Eosin Staining Microscopy Count":-0.5080310202,"Positive Finding Lymph Node Keratin Immunohistochemistry Staining Method Count":-0.3916789398,"Lymph Node(s) Examined Number":-0.5250612276,"Mutation Count":-0.0710643027,"Overall Survival (Months)":-0.2436468601,"Sample Initial Weight":2.6717943451,"TMB (nonsynonymous)":-0.0713319454,"ER Status IHC Percent Positive":1.6300260498,"HER2 ihc score":-0.2894793579,"PR status ihc percent positive":2.246833171,"American Joint Committee on Cancer Metastasis Stage Code_0":0.443859065,"American Joint Committee on Cancer Metastasis Stage Code_1":-0.1316093316,"American Joint Committee on Cancer Metastasis Stage Code_2":-0.4104083494,"American Joint Committee on Cancer Metastasis Stage Code_3":-0.0584538835,"Neoplasm Disease Lymph Node Stage American Joint Committee on Cancer Code_0":-0.6594341667,"Neoplasm Disease Lymph Node Stage American Joint Committee on Cancer Code_1":-0.1600349205,"Neoplasm Disease Lymph Node Stage American Joint Committee on Cancer Code_2":2.436597602,"Neoplasm Disease Lymph Node Stage American Joint Committee on Cancer Code_3":-0.0337099931,"Neoplasm Disease Lymph Node Stage American Joint Committee on Cancer Code_4":-0.3638553909,"Neoplasm Disease Lymph Node Stage American Joint Committee on Cancer Code_5":-0.4179314138,"Neoplasm Disease Lymph Node Stage American Joint Committee on Cancer Code_6":-0.1909727421,"Neoplasm Disease Lymph Node Stage American Joint Committee on Cancer Code_7":-0.0477002396,"Neoplasm Disease Lymph Node Stage American Joint Committee on Cancer Code_8":-0.1877568746,"Neoplasm Disease Lymph Node Stage American Joint Committee on Cancer Code_9":-0.2320080843,"Neoplasm Disease Lymph Node Stage American Joint Committee on Cancer Code_10":-0.2478825995,"Neoplasm Disease Lymph Node Stage American Joint Committee on Cancer Code_11":-0.1524099856,"Neoplasm Disease Lymph Node Stage American Joint Committee on Cancer Code_12":-0.2064062748,"Neoplasm Disease Lymph Node Stage American Joint Committee on Cancer Code_13":-0.0337099931,"Neoplasm Disease Lymph Node Stage American Joint Committee on Cancer Code_14":0.0,"Neoplasm Disease Lymph Node Stage American Joint Committee on Cancer Code_15":-0.127073347,"Neoplasm Disease Stage American Joint Committee on Cancer Code_0":-0.3028161911,"Neoplasm Disease Stage American Joint Committee on Cancer Code_1":3.4855779623,"Neoplasm Disease Stage American Joint Committee on Cancer Code_2":-0.0755497357,"Neoplasm Disease Stage American Joint Committee on Cancer Code_3":-0.0755497357, ...}'

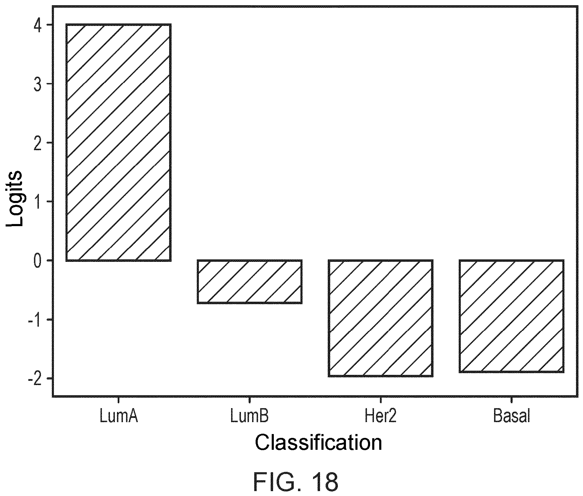

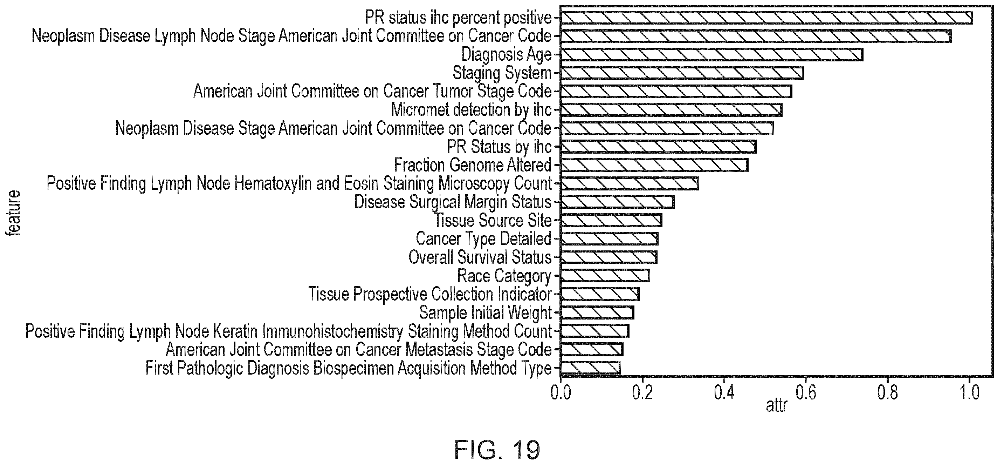

The resulting numerical vector is then used in the third ML model (after training) to generate the third output logits, which are illustrated in Figure 18. For explainability the third ML model uses Integrated Gradients. The attribution of each dimension in the input is calculated. Since a single raw feature can contribute multiple input values due to one-hot encoding, the contributions are summed for each feature to get the overall feature contribution. The absolute value of these contributions is taken and the features are ranked based on their importance to get explainability. The top contributing features for the subject are shown in Figure 19.

The third output logits are serialized and sent to the integration module for making the multi-modal prediction.

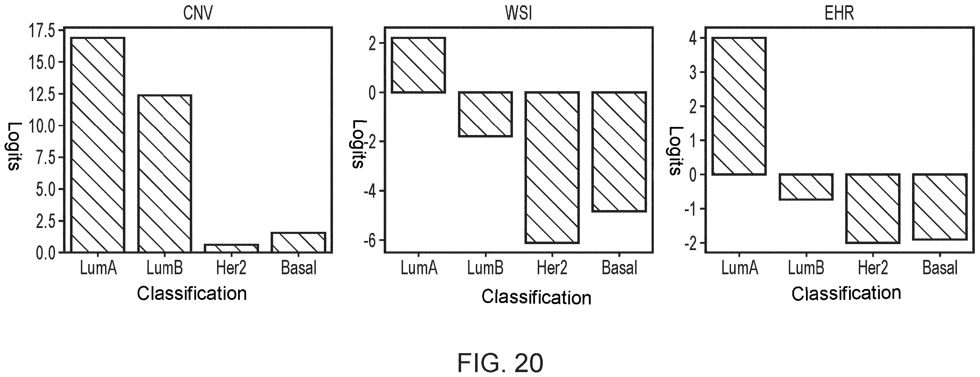

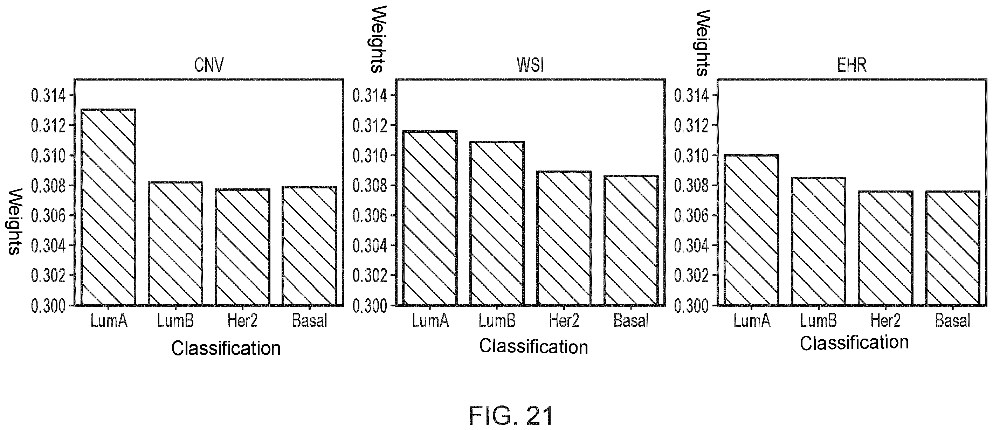

The first, second, and third output logits for the subject "tcga-a2-a0ev-01" are shown in Figure 20. For each modality and for each class, a weight is stored that was learned during the training process. The learned weights are shown in Figure 21.

The single modality outputs are fused following the previously described equation:

$$Lg_j = (1/3) * \sum(\alpha_{ij} * Lg_j^{(i)}), \text{ sum is from } i = 1 \text{ to } 3.$$

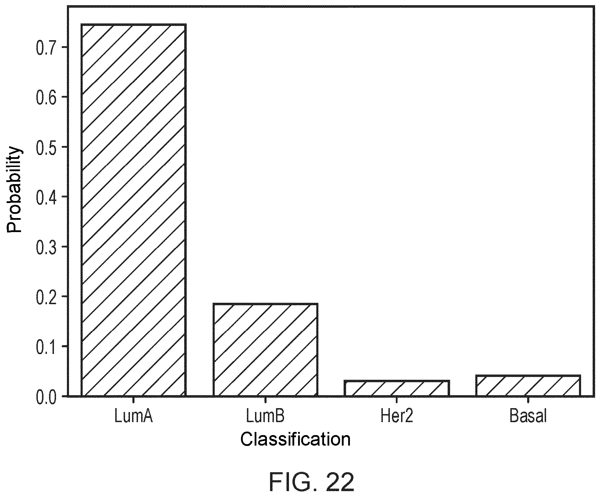

where $Lg_j^{(i)}$ is the jth element of the output of the ith modality (i.e. the logit for class/subtype j and modality i), $Lg_j$ is the jth element of the multimodal prediction logits, and $\alpha_{ij}$ are the weights. In other words, the weights are element-wise multiplied by the corresponding modality and corresponding class to produce rescaled model outputs. The mean is computed across the modalities. A Softmax function is applied to produce the multi-modal probability prediction. The final multi-modal probability output for the subject in this worked example is shown in Figure 22. The classification (subtype) is taken as the subtype with the highest probability, which in this case is Luminal A. This is the correct subtype according to the PAM50 ground truth classification.

Results

The methodology disclosed herein was trained and tested in line with the above description and results are discussed below. Specifically, the WSI, CNV, and EHR data from the TCGA-BRCA dataset was used for training and testing. As described above, instances that have any missing modality or do not have a PAM50 classification label are filtered out, and the number of included cases after filtration is 977 subjects. As also substantially described above, the data imbalance in the subtypes present in the training data was mitigated using one of three strategies: oversampling, stratified sampling and loss weighting. In oversampling, each class is resampled to have the same number of samples as the majority class; in stratified sampling, data is sampled from each class with probability inversely proportional to its size. In loss weighting, the loss of each class is re-scaled by a factor inversely proportional to its size in the dataset. For model evaluation, since the dataset is imbalanced, both accuracy and Area Under The Receiver Operating Characteristic Curve (AUROC) were used. For AUROC, the AUC per-class was calculated and then the average taken across classes. 10-fold cross validation was used for model evaluation and the average performance across the folds reported.
The best models after hyperparameter search were as following. For the first ML model, a backbone of SNN with 8192, 4096 and 2048 channel layers and a classification head with 512 and 128 channel hidden layers. We use balancing by oversampling for CNV data, and input is z-scored based on the training subset. For the second ML model, an Inceptionv3 backbone was used, with a classification head of 512 and 128 channel hidden layers with ReLU activation, instance normalization, dropout of 0.5, balancing by weighted loss and pooling by majority voting. For the third ML model, a vanilla MLP was used with 128 and 64 channel hidden layers, ReLU activation and instance normalization. For all single-modality experiments in breast cancer subtyping, Adam optimizer was used with default parameters.
Evaluation of the specific implementation described above revealed a performance of 76.88% accuracy and 0.8976 AUROC.
Disclosed herein are the following:

- A PAM50 breast cancer subtyping system that achieves improved performance by integrating CNV, EHR and WSI and hence does not depend on RNA for the multimodal prediction.
- A decentralized distributed implementation of the PAM50 breast cancer subtyping system that can operate on locally distributed data and using minimal communication.
- Distributed modules implementation that can make use of heterogeneous CPU/GPU resources in an efficient manner based on the modality complexity.
- An efficient multi-modal late fusion strategy that can operate in a distributed way using only other module's outputs without the need for central collection of data.
- A WSI module that can operate on low magnification WSI while providing competitive single modality and multimodal performance. Moreover, it can be used as a standalone single modality module or part of a multimodal system. The module can provide heatmaps to explain the input regions that mostly contributed to its decision.
- CNV, EHR and WSI modules with implementation that can provide explainability for each modality which helps the user to understand the system prediction.
- A CNV module with a simplified pipeline that can operate as a standalone module or as a part of a bigger healthcare application system. It can provide input attribution that shows the most contributing genes to its decision.
- An EHR module that can use different types of clinical data including numerical, ordinal and categorical features. It can operate as a standalone single-modality PAM50 classification system using EHR or as a component in a bigger healthcare system. The module can provide attribution to its input and rank the features based on their contribution to the decision.
- A PAM50 WSI segmentation pipeline that can segment tissue from background and reduce computations by filtering out WSI patches that are mostly background.

- Two balancing strategies: oversampling and loss weighting for enhancing the training performance of imbalanced PAM50 classes.
- A decentralized distributed implementation that can be deployed on heterogenous hardware of CPU/GPU configurations and operate on locally distributed modalities without the need for central data transfer. The support for heterogenous hardware allows for efficient resource utilization as more computationally expensive modules such as Digital histopathology processing can benefit from GPU acceleration, while less demanding modules can be run on CPU.
- An efficient Late fusion strategy that relies only on model output and hence suitable for decentralized multi-omics integration. Moreover, it has a competitive PAM50 multi-omics performance without relying on RNA data.

An advantage of the disclosed methodologies is that multi-omics data may reside on different systems and hence regulated by different laws and privacy terms which makes transferring them to a central system infeasible. Moreover, different data modalities have different computational constraints, so to achieve efficient utilization of computational resources, the disclosed methodologies are able to operate in a heterogenous hardware environment where it can utilize GPUs for computationally expensive modalities while using CPUs for less demanding workload.

Another advantage of the disclosed methodologies is the transparency of the classification system in clinical setting. An explainable system that is capable of giving the basis of its decision is important for doctors to trust and have confidence in the system operation.

The single modality modules/method steps are self-contained and can be used as a standalone PAM50 systems based on a single modality or integrated as a part of a healthcare application systems. The late fusion strategy may be used for other cross domains use cases where the downstream application can benefit from fusion of multi-modal data.

In general, healthcare applications are multi-modal problems that benefit from the integration of diverse data sources. With the accelerating explosion of the amount of data produced by different modalities related to healthcare, there is a dire need for multimodal machine learning methods that can digest and detect useful patterns from these data in a scalabale and flexible way. Disclosed herein is a scalable and loosely-coupled multimodal framework that integrates data from diverse modalities including genomics, clinical records and histopathology to perform breast cancer subtyping. The disclosed methodologies allow expansion to additional modalities (e.g. RNA-seq) and scaling up and down with minimal overhead and without the need for re-training existing modalities.

Classifying breast cancer into a subtype is a multi-faceted practice that depends on diverse sources of information including molecular data like Copy Number Variation (CNV) and Ribonucleic Acid sequencing (RNA-seq), histopathology images and Electronic Health Records (EHR). This is becoming more and more challenging for doctors and researchers, specially, with the explosion of the amount of information provided by the different relevant modalities. This calls for Machine Learning (ML) models that can help with digesting this flow of information, detecting patterns and drawing useful conclusions from the data.

A challenge that faces multimodal prediction is that, in clinical practice, there is a partial availability and variation of the number and type of modalities that can be utilized by the multimodal method. Different datasets and different downstream tasks can have different number and types of modalities available at any given time. Hence, there is a need for a scalable and flexible multimodal methods that can scale to a large number of modalities and can adapt to the change of the number and type of modalities efficiently and with minimum overhead. Scalability and flexibility of a multimodal method is very important in medical practice. Medical data change and the availability of modalities can vary over time. The disclosed methodologies are able to scale efficiently to adding or excluding modalities with minimal overhead.

In the above implementations, PAM50 classifications are used as the breast cancer subtypes, and in particular four are used, with "normal-like" being omitted, however "normal-like" may be included, and thus five subtypes may be considered. Other classifications/subtypes may be used.

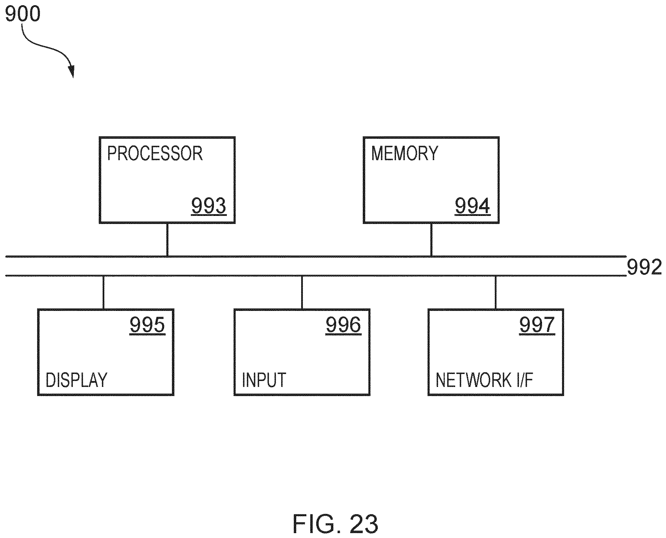

Figure 23 is a block diagram of an information processing apparatus 900 or a computing device 900, such as a data storage server, which embodies the present invention, and which may be used to implement some or all of the operations of a method embodying the present invention, and perform some or all of the tasks of apparatus of an embodiment. The computing device 900 may be used to implement any of the method steps described above, e.g. any of steps S10-S90 and/or S12-S60 and/or any processing of any of the modules 10, 20, 30, 60 described above.

The computing device 900 comprises a processor 993 and memory 994. Optionally, the computing device also includes a network interface 997 for communication with other such computing devices, for example with other computing devices of invention embodiments. Optionally, the computing device also includes one or more input mechanisms such as keyboard and mouse 996, and a display unit such as one or more monitors 995. These elements may facilitate user interaction. The components are connectable to one another via a bus 992.

The memory 994 may include a computer readable medium, which term may refer to a single medium or multiple media (e.g., a centralized or distributed database and/or associated caches and servers) configured to carry computer-executable instructions. Computer-executable instructions may include, for example, instructions and data accessible by and causing a computer (e.g., one or more processors) to perform one or more functions or operations. For example, the computer-executable instructions may include those instructions for implementing a method disclosed herein, or any method steps disclosed herein, e.g. any of steps S10-S90 and/or S12-S60 and/or any processing of any of the modules 10, 20, 30, 60 described above. Thus, the term "computer-readable storage medium" may also include any medium that is capable of storing, encoding or carrying a set of instructions for execution by the machine and that cause the machine to perform any one or more of the method steps of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing computer program code stored in the memory 994 to implement any of the method steps described herein, e.g. any of steps S10-S90 and/or S12-S60 and/or any processing of any of the modules 10, 20, 30, 60 described above. The memory 994 stores data being read and written by the processor 993 and may store CNV data and/or histopathology image data and/or medical data and/or WSI data and/or EHR data and/or weights of the ML models and/or learnable weights and/or output logits and/or training data and/or testing data and/or an output classification and/or input data and/or other data, described above, and/or programs for executing any of the method steps or processes described above.

As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the method steps and/or operations discussed herein, e.g. any of steps S10-S90 and/or S12-S60 and/or any processing of any of the modules 10, 20, 30, 60 described above. The processor 993 may be considered to comprise any of the modules 10, 20, 30, 60 described above. Any operations described as being implemented by a module may be implemented as a method by a computer and e.g. by the processor 993. The processor 993 may be considered as comprising a plurality of processors/computing devices.

The display unit 995 may display a representation of data stored by the computing device, such as a representation of the first ML model and/or a representation of the second ML model and/or a representation of the third ML model and/or a representation of a breast cancer classification and/or input data and/or GUI windows and/or interactive representations enabling a user to interact with the apparatus 900 by e.g. drag and drop or selection interaction, and/or any other output described above, and may also display a cursor and dialog boxes and screens enabling interaction between a user and the programs and data stored on the computing device. The input mechanisms 996 may enable a user to input data and instructions to the computing device, such as enabling a user to input any user input described above.

The network interface (network I/F) 997 may be connected to a network, such as the Internet, and is connectable to other such computing devices via the network. The network I/F 997 may control data input/output from/to other apparatus via the network. For example, computing devices similarly configured to device 900 may be configured to operate remotely together to implement the first, second, and third ML models and generate the weighted combination and ultimately the classification of the breast cancer. Other peripheral devices such as microphone, speakers, printer, power supply unit, fan, case, scanner, trackerball etc may be included in the computing device.

Methods embodying the present invention may be carried out on a computing device/apparatus 900 such as that illustrated in Figure 23. Such a computing device need not have every component illustrated in Figure 23, and may be composed of a subset of those components. For example, the apparatus 900 may comprise the processor 993 and the memory 994 connected to the processor 993. Or the apparatus 900 may comprise the processor 993, the memory 994 connected to the processor 993, and the display 995. A method embodying the present invention may be carried out by a single computing device in communication with one or more data storage servers via a network. The computing device may be a data storage itself storing at least a portion of the data.

A method embodying the present invention may be carried out by a plurality of computing devices operating in cooperation with one another. One or more of the plurality of computing devices may be a data storage server storing at least a portion of the data. The device 900 may be a computing device among a plurality of similarly configured computing devices, wherein the computing devices are together configured for carrying out any method steps described herein, e.g. any of steps S10-S90 and/or S12-S60 and/or any processing of any of the modules 10, 20, 30, 60 described above. For example, at least one said computing device may train and/or implement the first ML model, at least one said computing device may train and/or implement the second ML model, at least one said computing device may train and/or implement the third ML model, and at least one said computing device may train and/or implement the weights for computing the weighted combination and classifying a breast cancer. The processors and/or computing devices may be heterogeneous e.g. in terms of processing power.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The above-described embodiments of the present invention may advantageously be used independently of any other of the embodiments or in any feasible combination with one or more others of the embodiments.

## Claims

1. A computer-implemented method comprising:

   applying a first machine learning, ML, model on copy number variation, CNV, data relating to a subject to generate first output logits corresponding respectively to a plurality of breast cancer sub-types, wherein the first ML model has been trained to classify CNV data as corresponding to a sub-type of the plurality of breast cancer sub-types;
   applying a second ML model on histopathology image data relating to the subject to generate second output logits corresponding respectively to the plurality of breast cancer sub-types, wherein the second ML model has been trained to classify histopathology data as corresponding to a sub-type of the plurality of breast cancer sub-types;
   applying a third ML model on medical data relating to the subject, including information relating to a diagnosis of breast cancer in the subject, to generate third output logits corresponding respectively to the plurality of breast cancer sub-types, wherein the third ML model has been trained to classify medical data, including information relating to a diagnosis of breast cancer, as corresponding to a sub-type of the plurality of breast cancer sub-types;
   computing a weighted combination of the first output logits, the second output logits, and the third output logits; and
   based on the weighted combination, classifying the subject's breast cancer as a sub-type of the plurality of breast cancer sub-types.

2. The computer-implemented method as claimed in claim 1, wherein classifying the subject's breast cancer as a sub-type of the plurality of breast cancer sub-types comprises generating, based on the weighted combination, a probability distribution using a softmax function, and classifying the subject's breast cancer according to the probability distribution.

3. The computer-implemented method as claimed in claim 1 or claim 2, wherein computing the weighted combination of the first output logits, the second output logits, and the third output logits comprises using first weights, second weights, and third weights corresponding respectively to the first, second, and third output logits, wherein the first weights, second weights, and third weights each comprise weights corresponding respectively to the plurality of breast cancer sub-types.

4. The computer-implemented method as claimed in claim 3, wherein the first, second, and third weights have been trained according to a training process.

5. The computer-implemented method as claimed in claim 4, wherein the training process comprises:

computing a training weighted combination of first, second, and third training output logits, the first training output logits the result of applying the first ML model on training CNV data relating to a training subject, the second training output logits the result of applying the second ML model on training histopathology image data relating to the training subject, and the third training output logits the result of applying the third ML model on training medical data relating to the training subject, including information relating to a diagnosis of breast cancer in the training subject;

based on the training weighted combination, classifying the training subject's breast cancer as a sub-type of the plurality of breast cancer sub-types; and

comparing the classification with ground truth data indicating a ground-truth sub-type corresponding to the training subject and adjusting at least one of the first weights, the second weights, and the third weights based on the comparison.

6. The computer-implemented method as claimed in claim 3, wherein the subject is a training subject and the method comprises comparing the classification with ground truth data indicating a ground-truth sub-type corresponding to the training subject and adjusting at least one of the first weights, the second weights, and the third weights based on the comparison.

7. The computer-implemented method as claimed in any of claims 1 to 5, wherein the subject is a target patient and wherein the computer-implemented method comprises outputting a diagnosis of the target patient including the classifying of the subject's breast cancer as a sub-type of the plurality of breast cancer sub-types.

8. The computer-implemented method as claimed in any of the preceding claims, wherein the plurality of breast cancer sub-types comprises a plurality of PAM50 breast cancer sub-types.

9. The computer-implemented method as claimed in any of the preceding claims, comprising using a first at least one processor or computing device for applying the second ML model, the first at least one processor or computing device being comparatively larger and/or having a higher processing capacity than a second at least one processor or computing device used for any of:

applying the first ML model;
applying the third ML model;
computing the weighted combination; and
classifying the subject's breast cancer.

10. The computer-implemented method as claimed in any of the preceding claims, comprising using a plurality of processors and/or computing devices located in different geographical locations and/or in different medical facilities.

11. A computer program which, when run on a computer, causes the computer to carry out a method comprising:

applying a first machine learning, ML, model on copy number variation, CNV, data relating to a subject to generate first output logits corresponding respectively to a plurality of breast cancer sub-types, wherein the first ML model has been trained to classify CNV data as corresponding to a sub-type of the plurality of breast cancer sub-types;
applying a second ML model on histopathology image data relating to the subject to generate second output logits corresponding respectively to the plurality of breast cancer sub-types, wherein the second ML model has been trained to classify histopathology data as corresponding to a sub-type of the plurality of breast cancer sub-types;
applying a third ML model on medical data relating to the subject, including information relating to a diagnosis of breast cancer in the subject, to generate third output logits corresponding respectively to the plurality of breast cancer sub-types, wherein the third ML model has been trained to classify medical data, including information relating to a diagnosis of breast cancer, as corresponding to a sub-type of the plurality of breast cancer sub-types;
computing a weighted combination of the first output logits, the second output logits, and the third output logits; and
based on the weighted combination, classifying the subject's breast cancer as a sub-type of the plurality of breast cancer sub-types.

12. An information processing apparatus comprising a memory and a processor connected to the memory, wherein the processor is configured to:

apply a first machine learning, ML, model on copy number variation, CNV, data relating to a subject to generate first output logits corresponding respectively to a plurality of breast cancer sub-types, wherein the first ML model has been trained to classify CNV data as corresponding to a sub-type of the plurality of breast cancer sub-types; apply a second ML model on histopathology image data relating to the subject to generate second output logits corresponding respectively to the plurality of breast cancer sub-types, wherein the second ML model has been trained to classify histopathology data as corresponding to a sub-type of the plurality of breast cancer sub-types; apply a third ML model on medical data relating to the subject, including information relating to a diagnosis of breast cancer in the subject, to generate third output logits corresponding respectively to the plurality of breast cancer sub-types, wherein the third ML model has been trained to classify medical data, including information relating to a diagnosis of breast cancer, as corresponding to a sub-type of the plurality of breast cancer sub-types; compute a weighted combination of the first output logits, the second output logits, and the third output logits; and based on the weighted combination, classify the subject's breast cancer as a sub-type of the plurality of breast cancer sub-types.

13. A system comprising a plurality of computing devices configured to:

apply a first machine learning, ML, model on copy number variation, CNV, data relating to a subject to generate first output logits corresponding respectively to a plurality of breast cancer sub-types, wherein the first ML model has been trained to classify CNV data as corresponding to a sub-type of the plurality of breast cancer sub-types; apply a second ML model on histopathology image data relating to the subject to generate second output logits corresponding respectively to the plurality of breast cancer sub-types, wherein the second ML model has been trained to classify histopathology data as corresponding to a sub-type of the plurality of breast cancer sub-types; apply a third ML model on medical data relating to the subject, including information relating to a diagnosis of breast cancer in the subject, to generate third output logits corresponding respectively to the plurality of breast cancer sub-types, wherein the third ML model has been trained to classify medical data, including information relating to a diagnosis of breast cancer, as corresponding to a sub-type of the plurality of breast cancer sub-types; compute a weighted combination of the first output logits, the second output logits, and the third output logits; and based on the weighted combination, classify the subject's breast cancer as a sub-type of the plurality of breast cancer sub-types.

14. The system as claimed in claim 13, wherein the plurality of computing devices comprises a first at least one processor or computing device configured to apply the second ML model, the first at least one processor or computing device being comparatively larger and/or having a higher processing capacity than a second at least one processor or computing device configured to any of:

apply the first ML model;
apply the third ML model;
compute the weighted combination; and
classify the subject's breast cancer.

15. The system as claimed in claim 13 or claim 14, wherein the plurality of computing devices comprises a plurality of processors and/or computing devices located in different geographical locations and/or in different medical facilities.

**Amended claims in accordance with Rule 137(2) EPC.**

1. A computer-implemented method comprising:

applying (S10) a first machine learning, ML, model on copy number variation, CNV, data relating to a subject to generate first output logits corresponding respectively to a plurality of breast cancer sub-types, wherein the first ML model has been trained according to a first ML model training process to classify CNV data as corresponding to a sub-type of the plurality of breast cancer sub-types; applying (S20) a second ML model on histopathology image data relating to the subject to generate second output logits corresponding respectively to the plurality of breast cancer sub-types, wherein the second ML model has been trained according to a second ML model training process, distinct from the first ML model training process, to classify histopathology data as corresponding to a sub-type of the plurality of breast cancer sub-types; applying (S30) a third ML model on medical data relating to the subject, including information relating to a diagnosis of breast cancer in the subject, to generate third output logits corresponding respectively to the plurality of breast cancer sub-types, wherein the third ML model has been trained according to a third ML model training process, distinct from the first and second ML model training processes, to classify medical data, including information relating to a diagnosis of breast cancer, as corresponding to a sub-type of the plurality of breast cancer sub-types;

computing (S40) a weighted combination of the first output logits, the second output logits, and the third output logits; and

based on the weighted combination, classifying (S50) the subject's breast cancer as a sub-type of the plurality of breast cancer sub-types.

2. The computer-implemented method as claimed in claim 1, wherein classifying (S50) the subject's breast cancer as a sub-type of the plurality of breast cancer sub-types comprises generating, based on the weighted combination, a probability distribution using a softmax function, and classifying the subject's breast cancer according to the probability distribution.

3. The computer-implemented method as claimed in claim 1 or claim 2, wherein computing (S40) the weighted combination of the first output logits, the second output logits, and the third output logits comprises using first weights, second weights, and third weights corresponding respectively to the first, second, and third output logits, wherein the first weights, second weights, and third weights each comprise weights corresponding respectively to the plurality of breast cancer sub-types.

4. The computer-implemented method as claimed in claim 3, wherein the first, second, and third weights have been trained according to a training process.

5. The computer-implemented method as claimed in claim 4, wherein the training process comprises:

computing a training weighted combination of first, second, and third training output logits, the first training output logits the result of applying the first ML model on training CNV data relating to a training subject, the second training output logits the result of applying the second ML model on training histopathology image data relating to the training subject, and the third training output logits the result of applying the third ML model on training medical data relating to the training subject, including information relating to a diagnosis of breast cancer in the training subject;

based on the training weighted combination, classifying the training subject's breast cancer as a sub-type of the plurality of breast cancer sub-types; and

comparing (S90) the classification with ground truth data indicating a ground-truth sub-type corresponding to the training subject and adjusting at least one of the first weights, the second weights, and the third weights based on the comparison.

6. The computer-implemented method as claimed in claim 3, wherein the subject is a training subject and the method comprises comparing the classification with ground truth data indicating a ground-truth sub-type corresponding to the training subject and adjusting at least one of the first weights, the second weights, and the third weights based on the comparison.

7. The computer-implemented method as claimed in any of claims 1 to 5, wherein the subject is a target patient and wherein the computer-implemented method comprises outputting a diagnosis of the target patient including the classifying of the subject's breast cancer as a sub-type of the plurality of breast cancer sub-types.

8. The computer-implemented method as claimed in any of the preceding claims, wherein the plurality of breast cancer sub-types comprises a plurality of PAM50 breast cancer sub-types.

9. The computer-implemented method as claimed in any of the preceding claims, comprising using a first at least one processor or computing device for applying the second ML model, the first at least one processor or computing device being comparatively larger and/or having a higher processing capacity than a second at least one processor or computing device used for any of:

applying the first ML model;
applying the third ML model;
computing the weighted combination; and
classifying the subject's breast cancer.

10. The computer-implemented method as claimed in any of the preceding claims, comprising using a plurality of processors and/or computing devices located in different geographical locations and/or in different medical facilities.

11. A computer program which, when run on a computer, causes the computer to carry out a method comprising:

applying (S10) a first machine learning, ML, model on copy number variation, CNV, data relating to a subject to generate first output logits corresponding respectively to a plurality of breast cancer sub-types, wherein the first ML model has been trained according to a first ML model training process to classify CNV data as corresponding to a sub-type of the plurality of breast cancer sub-types;

applying (S20) a second ML model on histopathology image data relating to the subject to generate second output logits corresponding respectively to the plurality of breast cancer sub-types, wherein the second ML model has been trained according to a second ML model training process, distinct from the first ML model training process, to classify histopathology data as corresponding to a sub-type of the plurality of breast cancer sub-types;

applying (S30) a third ML model on medical data relating to the subject, including information relating to a diagnosis of breast cancer in the subject, to generate third output logits corresponding respectively to the plurality of breast cancer sub-types, wherein the third ML model has been trained according to a third ML model training process, distinct from the first and second ML model training processes, to classify medical data, including information relating to a diagnosis of breast cancer, as corresponding to a sub-type of the plurality of breast cancer sub-types;

computing (S40) a weighted combination of the first output logits, the second output logits, and the third output logits; and

based on the weighted combination, classifying (S50) the subject's breast cancer as a sub-type of the plurality of breast cancer sub-types.

12. An information processing apparatus (900) comprising a memory (994) and a processor (993) connected to the memory (994), wherein the processor (993) is configured to:

apply a first machine learning, ML, model on copy number variation, CNV, data relating to a subject to generate first output logits corresponding respectively to a plurality of breast cancer sub-types, wherein the first ML model has been trained according to a first ML model training process to classify CNV data as corresponding to a sub-type of the plurality of breast cancer sub-types;

apply a second ML model on histopathology image data relating to the subject to generate second output logits corresponding respectively to the plurality of breast cancer sub-types, wherein the second ML model has been trained according to a second ML model training process, distinct from the first ML model training process, to classify histopathology data as corresponding to a sub-type of the plurality of breast cancer sub-types;

apply a third ML model on medical data relating to the subject, including information relating to a diagnosis of breast cancer in the subject, to generate third output logits corresponding respectively to the plurality of breast cancer sub-types, wherein the third ML model has been trained according to a third ML model training process, distinct from the first and second ML model training processes, to classify medical data, including information relating to a diagnosis of breast cancer, as corresponding to a sub-type of the plurality of breast cancer sub-types;

compute a weighted combination of the first output logits, the second output logits, and the third output logits; and

based on the weighted combination, classify the subject's breast cancer as a sub-type of the plurality of breast cancer sub-types.

FIG. 1

FIG. 2

EP 4 687 143 A1

FIG. 3

FIG. 4

FIG. 5

CNV (23k)

8192

4096

2048

512

128

prediction

FIG. 6

A

B

C

FIG. 7

FIG. 8

FIG. 9

S10 — Apply first ML model on CNV data to generate first output logits

S20 — Apply second ML model on histopathology image data to generate second output logits

S30 — Apply third ML model on medical data to generate third output logits

S40 — Compute weighted combination of first to third output logits

S50 — Classify subject's breast cancer

S90 — Compare with true classification and adjust weights

FIG. 10

FIG. 11

FIG. 12

FIG. 13

FIG. 14

FIG. 15

FIG. 16

FIG. 17

FIG. 18

FIG. 19

FIG. 20

EP 4 687 143 A1

FIG. 21

EP 4 687 143 A1

FIG. 22

900

| PROCESSOR |
| 993 |

| MEMORY |
| 994 |

992

| 995 |
| DISPLAY |

| 996 |
| INPUT |

| 997 |
| NETWORK I/F |

FIG. 23

Europäisches
Patentamt

European
Patent Office

Office européen
des brevets

# EUROPEAN SEARCH REPORT

**Application Number**

EP 24 19 1385

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RAKTIM KUMAR MONDOL ET AL: "BioFusionNet: Deep Learning-Based Survival Risk Stratification in ER+ Breast Cancer Through Multifeature and Multimodal Data Fusion", ARXIV.ORG, CORNELL UNIVERSITY LIBRARY, 201 OLIN LIBRARY CORNELL UNIVERSITY ITHACA, NY 14853, 3 June 2024 (2024-06-03), XP091775445, * the whole document, in particular the abstract, sections 2.2, 2.3, 2.4 and figures 2, 3 * | 1-15 | INV. G16B40/20 G16H30/40 G16H50/20 |
| Y | CHEN RICHARD J. ET AL: "Pathomic Fusion: An Integrated Framework for Fusing Histopathology and Genomic Features for Cancer Diagnosis and Prognosis", IEEE TRANSACTIONS ON MEDICAL IMAGING, vol. 41, no. 4, 1 April 2022 (2022-04-01), pages 757-770, XP055940624, USA ISSN: 0278-0062, DOI: 10.1109/TMI.2020.3021387 Retrieved from the Internet: URL:http://xplorestaging.ieee.org/ielx7/42 /9745979/09186053.pdf?arnumber=9186053> * the whole document, in particular the abstract, section III * | 1-15 | |
| A | CAN CUI ET AL: "Deep Multi-modal Fusion of Image and Non-image Data in Disease Diagnosis and Prognosis: A Review", ARXIV.ORG, CORNELL UNIVERSITY LIBRARY, 201 OLIN LIBRARY CORNELL UNIVERSITY ITHACA, NY 14853, 25 March 2022 (2022-03-25), XP091186247, * the whole document * | 1-15 | |

-----

-----

-----

-/--

| | | | |
|---|---|---|---|
| TECHNICAL FIELDS SEARCHED (IPC) | | | |
| G16B G16H | | | |

The present search report has been drawn up for all claims

| Place of search | Date of completion of the search | Examiner |
|---|---|---|
| Munich | 9 January 2025 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RAKTIM KUMAR MONDOL ET AL: "hist2RNA: An efficient deep learning architecture to predict gene expression from breast cancer histopathology images", ARXIV.ORG, CORNELL UNIVERSITY LIBRARY, 201 OLIN LIBRARY CORNELL UNIVERSITY ITHACA, NY 14853, 2 May 2023 (2023-05-02), XP091498599, DOI: 10.3390/CANCERS15092569 * the whole document * ----- | 1-15 | |

**TECHNICAL FIELDS SEARCHED (IPC)**

The present search report has been drawn up for all claims

| Place of search | Date of completion of the search | Examiner |
|---|---|---|
| Munich | 9 January 2025 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SZEGEDY, C. et al.** Rethinking the inception architecture for computer vision. *Proceedings of the IEEE conference on computer vision and pattern recognition*, 2016, 2818-2826
- **K. SIMONYAN** ; **A. ZISSERMAN**. Very deep convolutional networks for large-scale image recognition. *arXiv preprint arXiv:1409.1556*, 2014
- **M. OQUAB et al.** Dinov2: Learning robust visual features without supervision. *arXiv preprint arXiv:2304.07193*, 2023
- Cancer Cancer Staging Handbooks. American Joint Committee
- International Classification of Diseases for Oncology
- **J. DEVLIN et al.** Bert: Pre-training of deep bidirectional transformers for language understanding. *arXiv preprint arXiv:1810.04805*, 2018
- **Y. LIU et al.** Roberta: A robustly optimized bert pretraining approach. *arXiv preprint arXiv: 1907.11692*, 2019
- **NETANELY, D.** Expression and methylation patterns partition luminal-A breast tumors into distinct prognostic subgroups. *Breast Cancer Research*, 2016, vol. 18, 1-16
- **DIEDERIK P. KINGMA** ; **JIMMY BA, ADAM**. A Method for Stochastic Optimization. *arxiv.org/abs/1412.6980*, 2017
- Imagenet: A large-scale hierarchical image database. **DENG, J. et al.** 2009 IEEE conference on computer vision and pattern recognition. Ieee, 2009, 248-255